(19) Europäisches Patentamt
European Patent Office
Office européen des brevets

(11) EP 1 831 698 B1

(12) **EUROPEAN PATENT SPECIFICATION**

(45) Date of publication and mention of the grant of the patent:
**27.01.2010 Bulletin 2010/04**

(21) Application number: **05804993.3**

(22) Date of filing: **23.12.2005**

(51) Int Cl.:
***G01N 33/58*** *(2006.01)*

(86) International application number:
**PCT/GB2005/005093**

(87) International publication number:
**WO 2006/067517 (29.06.2006 Gazette 2006/26)**

# (54) ANALYTICAL COMPOSITION AND METHOD

ANALYTISCHE ZUSAMMENSETZUNG UND VERFAHREN

COMPOSITION D'ANALYSE ET PROCEDE ASSOCIE

(84) Designated Contracting States:
**AT BE BG CH CY CZ DE DK EE ES FI FR GB GR HU IE IS IT LI LT LU LV MC NL PL PT RO SE SI SK TR**

(30) Priority: **24.12.2004 GB 0428317**

(43) Date of publication of application:
**12.09.2007 Bulletin 2007/37**

(73) Proprietor: **The Robert Gordon University**
**Aberdeen, AB10 1FR (GB)**

(72) Inventors:
- **POLLARD, Patricia Mary**
  **Aberdeen AB15 6BH (GB)**
- **OFFICER, Simon,**
  **Robert Gordon University**
  **Aberdeen AB10 1FR (GB)**
- **HUNTER, Catherine,**
  **Robert Gordon University**
  **Aberdeen AB10 1FR (GB)**
- **PRABHU, G., Radharishna,**
  **Robert Gordon University**
  **Aberdeen AB10 1FR (GB)**
- **WILSON, Theresa,**
  **c/o The Robert Gordon University**
  **Aberdeen, Aberdeenshire, AB10 1FR (GB)**

(74) Representative: **Peter, Kenneth William et al**
**Murgitroyd & Company**
**165-169 Scotland Street**
**Glasgow G5 8PL (GB)**

(56) References cited:
**WO-A-00/55630** **FR-A- 2 846 647**
**US-A1- 2003 119 207** **US-A1- 2004 171 076**

- **HARMA HARRI ET AL: "Europium nanoparticles and time-resolved fluorescence for ultrasensitive detection of prostate-specific antigen" CLINICAL CHEMISTRY, vol. 47, no. 3, March 2001 (2001-03), pages 561-568, XP002370440 ISSN: 0009-9147**
- **DEJNEKA M J ET AL: "Rare earth-doped glass microbarcodes" PROCEEDINGS OF THE NATIONAL ACADEMY OF SCIENCES OF USA, NATIONAL ACADEMY OF SCIENCE, WASHINGTON, DC, US, vol. 100, no. 2, 21 January 2003 (2003-01-21), pages 389-393, XP002323047 ISSN: 0027-8424**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 831 698 B1

Printed by Jouve, 75001 PARIS (FR)

## Description

### Field of the Invention

The present invention relates to an analytical composition including a detectable marker. The invention also relates to an analytical method for detecting the marker.

### Background to the Invention

Analytical markers are used in assays to bind to or otherwise indicate the presence of target molecules in a sample being assayed. Existing markers are used to label antibodies or nucleic acid strands etc that bind to the target molecules of interest. The presence of chemical and biochemical markers (e.g. fluorophores, radioactive isotopes etc.) attached to the target-binding molecules indicates the presence of the target, and the amount of marker present can optionally be quantified by known techniques. Markers are often referred to as taggants, probes, labels, or tags.

Existing techniques to detect fluorescent markers are well known. Fluorophores emit light when excited by radiation of a particular wavelength. However, known fluorescent markers have the disadvantage that they generally have very broad spectra, which limits the number of markers that can be assayed at one time, and when there are several different binding events occurring in a single sample, distinguishing between them becomes more difficult.

A Europium nanoparticle is described in Clin Chem Härmä et al. 47 (3): 561, and its use in time resolved fluorescence for ultrasensitive detection of prostate-specific antigen is discussed. The particle is of polystyrene to which a europium chelate and streptavidin is applied.

Micrometer-sized glass barcodes containing a pattern of different fluorescent materials are described in Proceedings of the National Academy of Sciences of the United States of America, Vol. 100, No. 2 (Jan. 21, 2003), pp. 389-393, Matthew J. Dejneka, *et al*. Rare earth ions are introduced to an alumino-silicate glass that was melted, cast, annealed and drawn into canes that were suitably arranged so as to form the desired bar code pattern, and then drawn into ribbons from which individual barcodes were formed. Particles of such glass are also described in US 2004/0171076A1 (Matthew J. Dejneka, et al) and proposed for use as bead carriers, supports and labels in bioassays.

### Summary of the Invention

According to a first aspect of the present invention there is provided an optically detectable analytical composition comprising glass particles of less than 20μm, the said particles consisting of a glass carrier incorporating a rare earth dopant, and, bound to the carrier, at least one of a biological binding agent selected from biomolecules, and macromolecules, or a chemical linker suitable to bind to a biological binding agent, said linker providing a means of modifying the surface of the glass carrier to render it receptive to attachment of a biomolecule or macromolecule, wherein the glass is a borosilicate glass.

The glass particles may be less than 5μm.

The glass particles may be beads typically having a size range of a few microns.

The borosilicate based glass carrier, may have a composition comprising $SiO_2$; NaO; CaO; MgO; $Al_2O_3$; FeO and/or $Fe_2O_3$; $K_2O$ and $B_2O_3$ wherein the rare earth dopant is preferably a lanthanide.

Optionally the glass has a composition of $SiO_2$ 51.79 wt%; NaO 9.79 wt%; CaO 7.00 wt%; MgO 2.36 wt%; $Al_2O_3$ 0.29 wt%; FeO, $Fe_2O_3$ 0.14 wt%; $K_2O$ 0.07 wt% and $B_2O_3$ 28.56 wt%.

The dopant is typically a lanthanide. The rare earth dopants may be selected from Europium, Dysprosium, and Terbium.

Optionally a plurality of rare earth dopants is used. One or more of these different rare earth dopants may have intrinsic fluorescent emissions that are visible to the unaided human eye and one or more may have intrinsic fluorescent emissions that are invisible to the unaided human eye, for example infrared or ultra-violet fluorescent emissions.

Optionally, the combined effect of the carrier and the rare earth dopant is such as to cause the composition to emit light that is visible by the unaided eye, for example in the range of 390-700nm.

Typically the composition can be excited by highly selective, high intensity visible light and the resultant emission can be detected in the visible region.

The chemical linker when attached to the carrier may exhibit polar properties. For example the chemical linker may be a polar group, such as silane, or a non-polar group *per se*, such as polystyrene, which when attached to the carrier exhibits polar properties.

The linker need not be chemically linked to the carrier - it may be a coating for example a polystyrene coating.

Preferably silane is utilised when the biological binding agent comprises a nucleic acid.

Preferably polystyrene is utilised when the biological binding agent comprises proteins such as antibodies.

Optionally the chemical linker attached to the carrier may exhibit a negative charge. For example, amino-silane,

mercapto-silane.

Alternatively the chemical linker attached to the carrier may exhibit a positive charge. For example, a positive charge may be achieved by treating the carrier with streptavidin.

Optionally the chemical linker comprises an oxygen atom.

The chemical linker may be a modified portion of the carrier. For example the chemical linker may comprise a thiol group, a carboxylic group (preferably activated carboxylic group), an iodo-acetamide group or a maleimide group.

Preferably the composition comprises the biological binding agent.

According to a second aspect of the invention there is provided a method of analysing a target in a sample, the method comprising:

(a) providing an analysis composition as in the first aspect, wherein a biological binding agent conjugated to a particle is adapted or chosen to bind to the target;

(b) exposing the sample and the biological binding agent to each other;

(c) separating (i) the biological binding agent which has bound to any target from (ii) the biological binding agent which has not bound to any target;

(d) and, to produce emissions from the rare earth element, illuminating at least one of (i) the biological binding agent which has bound to any target and (ii) the biological binding agent which has not bound to any target;

(e) detecting any emission from the sample illuminated in step (e); and,

(f) relating the detected emission to any feature of the target including its presence or absence in the sample.

Each sample can typically be illuminated with multiple wavelengths using an array of different detection filters and photomultipliers so that the emission at each pre-selected wavelength can be determined.

In certain embodiments the sample may be provided such that any target is immobilised. For example the sample may be provided on a membrane so that a target, such as an antigen, is essentially fixed or immobilised.

When the biological binding agent and sample are exposed to each other in step (c) the biological binding agent will bind to any of the immobilised target and thus be immobilised itself.

Separating the unbound biological agent according to step (d) may be performed by washing the sample since unbound biological binding agent will wash away whilst bound biological binding agent will be immobilised since it is bound to the target.

Thus in such embodiments, an emission from the bound sample as per step (f) will only be detected in the presence of the target. In such embodiments, the invention according to a second aspect of the invention is a method to detect the presence or absence of the target in the sample and optionally the amount of the target in the sample.

In other embodiments, the separating step (d) may be performed by gel-electrophoresis. The unbound biological binding agent will travel further through the gel than any bound biological binding agent.

Embodiments of the invention may be used to determine the size of the target, for example in DNA fragment analysis. Thus said feature in step (g) is the size of the target. In such an embodiment, the DNA is present but its size is unknown and embodiments of the present invention can be used to determine its size. The method according to such embodiments need not provide information about the exact molecular size of the target.

The biological binding agent may be one or more nucleotides, for example a chain of nucleotides i.e. a nucleic acid and the target may be a complementary nucleotide/nucleic acid. Nucleic acids include DNA, RNA, oligonucleotides, alleles and genes.

The binding agent can typically bind specifically to a target molecule to be identified or quantified.

The binding agent can be a protein such as an antibody, optionally a monoclonal antibody, but polyclonal antibodies can also be useful in this aspect. Non-antibody ligands and chelating agents can also be useful, and nucleic acid based binding agents such as strands of DNA or RNA adapted to hybridise to the target nucleic acid sequences can also be used. More than one target molecule can be bound, and in some embodiments of the invention, the carrier bears a combination of different binding agents. However, in most embodiments, a single species of carrier with a specific fluorescent signature bears a single species of binding agent, e.g. a specific antibody adapted to bind only to a specific target molecule, so that the fluorescent signature of the carrier can be bound with the presence (and optionally the amount) of the specific target molecule. One advantage of binding a specific species of carrier (with one fluorescent signature) to one antibody, and a second carrier (with a second fluorescent signature) to another antibody, is the possibility of simultaneous multianalyte immunoassays for each target in the same sample. The rare earth (RE) elements permit highly sensitive fluorescence detection in discrete bands to indicate the binding of the two antibodies to their respective

target molecules in the sample being tested. A larger number of antibodies or ligands can be attached to these beads due to their large surface area, thereby increasing the detection limit above conventional binding assays.

The antibodies or other binding agents can be attached to the beads over the glass surface of the bead, and the beads can be dispersed in the analyte. A set of standard protocols, specific to the surface and ligands can be used for the binding process. Silanisation of the glass beads is one option. It is possible to achieve full surface cover over the beads with antibodies. A biological conjugate (e.g. an antigen) can attach to these antibodies with high specificity. Non-specific binding can be avoided by suitably blocking the empty sites on the bead. The unbound antigen can be removed by washing process. In a similar fashion, this can be extended for nucleic acid analysis using the same carrier beads.

Different biological probes each attached to a different multi-RE doped carrier beads can be used. Advantages with this method are the smaller diffusion lengths for the biomolecules & faster detection of large number of interactions in a small volume. Multi-spectral encoded beads can be made by incorporating rare-earth ions, with spectrally sharp absorption and fluorescence spectra, in suitable host material. These beads along with a suitable detection system can be used for labelled detection of biological interactions.

The method may include the step of conducting a hybridisation, such as a northern blot or a southern blot.

The method may be used to conduct fragment analysis of nucleic acids, such as DNA.

The biological binding agent may be a protein such as an enzyme, antibody, antigen etcetera.

One of the biological binding agent and target may be an antibody and the other of the biological binding agent and target may be an antigen

One of the biological binding agent and target may be a cellular species and the other may be a protein such as an enzyme, antigen, receptor etcetera.

The binding agent can itself be labelled e.g. with a conventional fluorophore, such as fluorescein or rhodamine, typically one that emits radiation at a wavelength different from the RE dopant.

A microscopic detection system with an option to spectrally resolve the signature from beads is preferred to read the fluorescent signature from the carrier beads. An X-Y scanning stage attached to this system can provide data collection from all beads. The beads can be identified from the spectral signature. A microscopic detection system is preferred, optionally comprising a time-resolved fluorometer, intrinsically fluorescent lanthanide doped beads and microparticles as the solid phase.

It will be understood already that a rare earth dopant has an intrinsic set of electronic energy levels, and that interaction between a carrier and the dopant is such that these intrinsic energy levels typically change when the dopant is incorporated into the carrier. For example, when the dopant is incorporated into a glass, new bonds are formed in the doped glass, thus altering the electron arrangement and hence the energy levels of absorption and fluorescent emission. Altering the rare earth dopant and/or dopant chelate and/or the composition of the carrier changes these energy levels and hence the observed fluorescent fingerprint of the composition.

The carrier in which the rare earth dopant is embedded can readily be produced in a variety of formats, e.g. microbeads or fibres. Alternatively they may be an integral part of the polymer matrix forming a product.

Due to the very discrete fluorescent wavelength of a carrier doped with a rare earth element, multiple carriers can be used (or a single carrier doped with multiple rare earth elements), each prepared to have a different pre-selected emission wavelength, so that a profile comprising multiple wavelengths can be provided in a single carrier without the different wavelengths overlapping each other.

The carrier doped with the rare earth ion has a new energy level profile that allows transitions different to those allowed by either the rare earth element or the un-doped carrier.

The new energy profile is particularly advantageous for identification purposes because it provides narrow emission at wavelengths not naturally found in either the rare earth element or the un-doped carrier. These narrow emissions can be used as part of an identification marker.

In a preferred embodiment, the markers have different concentrations of dopant, so that the intensities of the pre-selected wavelength emissions are different. By virtue of this feature, the relative emission intensity of different pre-selected wavelengths can be used as an additional identifying feature. For example, one pre-selected wavelength intensity may be 100%; another pre-selected wavelength intensity 50%; a third pre-selected intensity 25% and a fourth pre-selected intensity 5%. More or less than four wavelengths can be used.

In one embodiment, the emission from each marker decays over a different time period. By virtue of this feature, the time over which emission occurs for a particular wavelength can be used as part of an identification profile.

Optionally, the composition is illuminated using a pulsed laser or LEDs and optionally an illumination filter for ensuring that only a narrow band of wavelengths illuminate the item.

Typically the emissions from the doped beads are passed through a detection filter to filter out all wavelengths except the pre-selected wavelength, and supplied to a photomultiplier to detect the intensity of light passing through the detection filter.

An extension of the hybridised binding agent may be performed.

An embodiment of the invention will now be described by way of example only and with reference to the

accompanying drawings, in which:

Figure 1 is a schematic view of a detector system for analysing a fluorescent signal produced from a composition of the invention;

Figure 2 shows the absorption spectra of Eu-doped glass beads;

Figure 3 shows the absorption spectra of un-doped blank glass beads;

Figure 4 shows the fluorescence spectrum of un-doped blank glass;

Figure 5 shows the fluorescence spectrum of un-doped blank glass in the visible spectrum;

Figure 6 shows the fluorescence spectrum of 3%Eu-doped glass;

Figure 7 shows a typical laser pulse profile at 465nm;

Figure 8 shows a typical fluorescence signal pulse from 3% Eu-doped beads exposed to a laser pulse at 465nm;

Figure 9 shows a wet sieving apparatus;

Figure 10 shows a particle size distribution of a sample of glass beads for use in an embodiment of the invention;

Figs. 11a- 11d show schematic diagrams of the principle steps of an assay of one embodiment of the invention;

Figure 12 shows a schematic diagram of a southern blot analytical technique used in accordance with one embodiment of the present invention;

Figure 13 shows a reaction scheme of an Acrydite™ modified material with thiol groups, leading to formation of a stable thioether bond; and,

Figure 14 is a reaction scheme showing the connection between a thiol modified biological binding agent with a carrier.

Embodiments of the present invention provide an optically detectable analytical composition comprising a glass bead incorporating a rare earth dopant and a binding agent which is bound to the glass bead optionally via a chemical linker.

The glass bead/rare earth dopant produces an identifiable spectrum when illuminated. (This may be identified by wavelength or by intensity.) In particular, the spectrum produced is of a very narrow range compared with known fluorophores.

As explained in more detail below, a number of such beads may be used with different binding agents. The different binding agents can be chosen to bind to a number of different targets in a sample. After the beads have been exposed to a sample, the composition can be scanned and a combined spectrum of the different spectra emitted by the different beads interpreted to determine the presence of scores of different targets in one assay.

Existing fluorophores can only be mixed with, for example three or, at most, four other dopants. If more are used the combined spectrum produced cannot be interpreted because of the overlapping of the wide bands produced.

The following detailed description provides information on how to prepare and modify glass for use in the composition and methods as defined by the claims.

## Carrier

Several methods for doping glass compositions with the selected fluorescent rare earth ions can be employed. In one method, test samples of doped glass are prepared by the incorporation of the rare earth ions into the batch composition using the appropriate metal salt. The glass was prepared by heating the batch in a platinum crucible to above the melting point of the mixture. In another method, existing standard glass samples are powdered and mixed with solutions of the fluorescent ions. The glass is lifted out of the solvent washed and then oven dried.

A glass comprising $SiO_2$ 51.79 wt%; NaO 9.79 wt%; CaO 7.00 wt%; MgO 2.36 wt%; $Al_2O_3$ 0.29 wt%; FeO, $Fe_2O_3$ 0.14 wt%; $K_2O$ 0.07 wt% and $B_2O_3$ 28.56 wt% can be made by ball milling soda lime beads (100μm) for 5 minutes to create a powder to help melting and mixing. Then 5g of the crushed soda lime beads, 2g of the $B_2O_3$ and 3mol% of

the rare earth dopant, for example Europium, Dysprosium and Terbium but also others, are ball milled together for, e.g. 3 minutes. The resulting powder is then put in a furnace and heated up to 550°C. It is left in the furnace at this temperature for about 30 minutes, to ensure that the boric oxide is completely melted. The temperature is increased to 900°C, 1000°C and then to 1100°C for 1 hour at each stage to produce a homogeneous melt. The temperature is optionally increased to 1250°C as a final step, and the molten glass is then poured into a brass mould, which is at room temperature, which quenches the glass to form a transparent, bubble free borosilicate glass, doped with rare earth ion. Optionally the brass mould can be heated to reduce the possibility of cracking during the pouring step.

The peak emission wavelength for fluorescent emission in the marker depends on the energy levels of the final rare earth doped glass. Altering the weight percentage of the network modifier oxides within the glass matrix will change these levels and hence change the observed peak fingerprint. Likewise, where two or more rare earth dopants are used, varying the ratios, by mole percentage, of these changes the fluorescence intensity in the detected signal. Peak intensities can be used as part of the encoding scheme and so by varying the dopant levels, there is provided an opportunity to provide even more signature options.

To crush the glass down to a fine powder a Glen Creston ball mill with agate vial and zirconia balls were used. Initial sieving down to 45μm was achieved using a standard 45μm sieve and sieve shaker. Below this size required further work due to problems caused by agglomeration and caking effects that hinder the passage of powder through conventional sieves.

The wet sieving technique was adapted from Mullin [2] and a diagram of the experimental set up shown in Fig 9. The process involved placing the sieved sample (1g) onto the sieve before lowering the sieve into a beaker of acetone so that the acetone was 1cm above the immersed sieving surface. The beaker was then placed into an ultrasonic bath and sonicated for 2 minutes. A copper wire was used to hook onto the sides of the beaker to hold the sieve in place.

The sieved sample became dispersed in the acetone below the sieve. Therefore the majority of acetone could be decanted off (after allowing time for the sample to settle) before allowing the rest to evaporate to dryness. This process can be used to produce samples below 5μm.

As an alternative to the wet sieving method described above, sonic sifters (e.g. from Endecotts) can also be useful as they can allow a dry sieving process down to single micron particle size. The method of sieving is by a variable vertical column of air that oscillates through a sieve or set of sieves. The motion of the air alternately lifts the sample and then assists it through the sieve apertures. A vertical mechanical pulse may also be applied to the sieves at regular intervals to break down any clustered particles and help eliminate any blocking of the apertures.

Samples of approximately 1g were placed on the sieve (10μm sieve) and run in the sonic sifter for periods of 9 minutes. This process was repeated until a sufficient quantity was produced. The resultant powder was white in comparison with the wet sieving powder, which was light brown in appearance. This produced much higher quantities of sample in a much shorter time. The purity of the sample was also evident with no sign of contamination caused by the sieving method.

To determine the particle size of the crushed samples, a Malvern Mastersizer/E was used. A 0.1 % solution of sodium hexametaphosphate (calgon) was used as a dispersion liquid to disperse the sample in the sample cell to allow an averaged value of particle size to be calculated. The 100mm focusing lens was used to measure the size range of 0.5-180μm. The quantity of sample added was determined by the software program on the computer attached to the instrument, which gave an indication of the optimum amount as the sample was added to the cell.

The particle size analyser distribution results are shown in Fig 10 and a table of the important results is shown in Table 1:

Table 1

| | |
|---|---|
| D[v,0.5] (divides distribution exactly in half | 5.72 μm |
| D[4.3] (volume mean diameter | 5.86 μm |
| D[v,0.1] (10% is below this value) | 0.80 μm |
| D[v,0.9] (90% is below this value) | 10.78 μm |
| 88.1% is under | 10.3 μm |

The computer program for the particle size analysis gives the data in various forms depending on how it worked out the size. The terms on the left hand side relate to the following [3]:

D [v,0.5] - Volume median diameter. This figure has 50% of the distribution above and 50% below this value. It divides the distribution exactly in half.

D [4,3] - Volume mean diameter. This is the diameter of the sphere that has the same volume as an ideal sphere.

$$D[4,3]= \Sigma d^4/\Sigma d^3$$

D [v,0.9], D [v,0.1] - These are 90% and 10% cut-offs respectively for the distribution. Where D [v,0.9] has 90% of the distribution below this value and D [v,0.1] has 10% of the distribution below this value.

The particle size analysis concluded that the majority of the sample collected after the sonic sifter procedure was under 10um.

The fluorescent signatures of blank beads were verified using a Perkin Elmer Lambda 2 UV/Vis Spectrophotometer. Settings selected to determine absorption spectra are shown in table 2.

Table 2

| | | | |
|---|---|---|---|
| Start | 900nm | Ord | A |
| End | 190nm | Speed | 960 |
| Int | 1 | Smooth | 2 |
| Ncycle | 1 | Lamps | 3 |

Each sample was inserted into a quartz cuvette to minimise the background signal. The data was then saved in a format that could be used in Microsoft Excel where the axis could be altered to achieve the optimum spectrum.

To determine the fluorescence spectra for each sample they were analysed using the Perkin Elmer LS50B Luminescence Spectrometer. Typical settings used to produce a 3D spectrum of the samples are shown in table 3.

Table 3

| ***Emisson Scan Range Parameters*** | | | | | |
|---|---|---|---|---|---|
| Start (nm): | 320 | End (nm): | 800 | Excitation (nm): | 300 |
| Ex Slit (nm): | 7.5 | Em Slit (nm): | 9 | Scan Speed (nm/min): | 1500 |
| 3D Scan Range Parameters | | | | | |
| Number of Scans: | 400 | | | Excitation Increment (nm): | 1 |

**Chemical Linker**

Before being conjugated to the binding agent for binding to the target, the glass bead carrier can be treated with a suitable chemical linker such as a coating to enhance conjugation of the binding agent with the carrier. In certain embodiments, the binding agent may first be chemically joined with the chemical linker and then the combined molecule attached, chemically or otherwise, to the glass bead.

For other embodiments the chemical linker may be a modified surface of the glass bead.

Some examples of treating the glass surface and/or conjugating the binding agent onto the glass bead are detailed below.

**Example 1 - Silanisation**

Preferably silanisation is used to attach nucleic acids to the glass beads. A method for silanisation of the glass beads is given below.

- Clean glass trays by rinsing with double distilled water before using. The silane tray should then be rinsed with 95% EtOH, and the acetone tray should be rinsed with acetone.
- Sonicate the glass beads in acetone for 10 minutes.
- After the acetone sonication, wash glass beads in the double distilled water tray at least twice.

- Sonicate the glass beads in 0.1 M NaOH for 10 minutes.
- Make the following silane solution under a hood:
- REAGENTS- Add:
- 95% EtOH 350 mL
- Glacial Acetic Acid 42 uL
- Glycidoxypropyltrimethoxy silane 11 mL
- After the NaOH sonication, wash beads in the double distilled water tray at least twice.
- Sonicate the beads in silane solution for at least 3 minutes.
- Wash the beads in the 100% EtOH tray.
- Dry with pre-purified nitrogen gas. Store in 100C oven. Wait at least 2 hours before using beads.

**Example 2 - Silanising oligonucleotides to glass beads.**

In this example the binding agent is first attached to the silane-containing group. The combined molecule is then attached to the glass bead.

Unmodified glass beads are first cleaned by ultrasonication for 30 minutes, followed by immersion in 10% NaOH for 30 minutes, then three washes in deionised water and one of distilled water. Beads are left to air-dry overnight.

The silanising protocol generally follows that described by Kumar *et al* (2000)[4], with some modifications. Generally, 5 nmol of the binding agent, that is 5' -thiol-modified oligonucleotides are reacted with the linker that is, 5nmol mercaptosilane (3-Mercaptopropyl-trimethoxysilane) in 30mM sodium acetate buffer (pH 4.3) for two hours at room temperature, see reaction scheme 1.

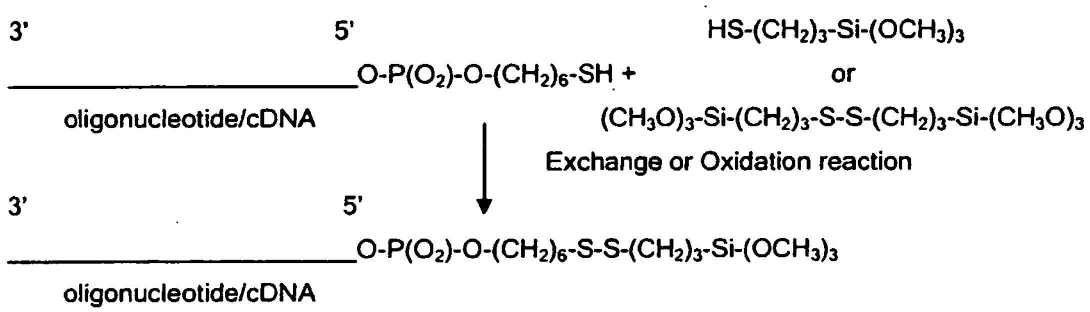


During this process, the oligonucleotides are chemically modified (silanised). Glass beads are then suspended in the minimum volume of silanised oligonucleotides. The beads are incubated in a humidified chamber (37°C for 30 minutes). The glass beads are then incubated (50°C for 10 minutes).

The binding agent with attached oligonucleotides is thus formed. Any unbound oligonucleotides are removed by immersion for 30 seconds in boiling distilled $H_2O$.

Alternative reaction schemes 2 and 3 are shown below.

3' 5'

__________________ $O-P(O_2)-O-(CH_2)_9-O-P(O_2)-O-NH-(CH_2)_3-NH-C(O)C(CH_3)=CH_2$

oligonucleotide/cDNA +

$H_2C=C(CH_3)CO_2(CH_2)_3Si(OCH_3)_3$

N,N,N',N'-Tetramethylethylenediamine
and Ammonium persulphate

3' 5'

__________________ $O-P(O_2)-O-(CH_2)_9-O-P(O_2)-O-NH-(CH_2)_3-NH-C(O)-C(CH_3)-CH_2-$

oligonucleotide/cDNA $(H_3CO)_3-Si-(CH_2)_3-C(O)-O-C(CH_3)-CH_2-$

**Reaction Scheme 2 Conjugating acrylic-labelled nucleic acids to acrylic-silane by polymerisation**

3' 5'

__________________ $O-P(O_2)-O-(CH_2)_6-SH + H_2N-(CH_2)_3-Si-(OCH_3)_3$

N-Succinimidyl-3-(2-pyridyldithiol)-propionate
or
Succinimidyl-6-(iodoacetylamino)-hexanoate

3' 5'

__________________ $O-P(O_2)-O-(CH_2)_6-S-S-(CH_2)_2-C(O)-NH-(CH_2)_5-Si-(OCH_3)_3$

oligonucleotide/cDNA

or

3' 5'

__________________ $O-P(O_2)-O-(CH_2)_6-S-CH_2-C(O)-NH(CH_2)_5-C(O)-NH-(CH_2)_3-Si(OCH_3)_3$

oligonucleotide/cDNA

**Reaction Scheme 3 Conjugating thiol-labelled nucleic acids to amino silane using a heterobifunctional cross linker.**

Example 3 Thioether linkage

As shown in Figure 13, the surface of the glass bead can be modified to provide a thiol. The binding agent (in this case DNA) modified with Acrydite™ is attached to the glass surface.

**Example 4**

As shown in Figure 14, the surface of the glass bead is treated to provide an amino-silane derivatised solid support (A) which is reacted with succinimidyl 4- [maleimidophenyl] butyrate (SMPB) in order to form a connection with the binding agent (C), which in this instance is a thiol modified oligonucleotide.

**Example 5** - Example of method for hybridisation with oligonucleotides with attached glass bead.

In this example using the technique of hybridising, complementary labelled oligonucleotide are used to bind

with the target oligonucleotides which have been separated by gel electrophoresis.

The technique of southern blotting or dot blotting or slot blotting is used to separate different fragments of DNA. Fig. 12 shows a suitable scheme comprising gel electrophoresis apparatus 10 with attached power supply 12, a membrane 14, dish 15, and scanner 18.

Oligonucleotides can be pre-hybridised in an oven at 60 or 65°C in hybridisation solution for at least 3 hours, to which no probe (taggant-labelled DNA) has been added.

The unlabelled oligonucleotides or DNA fragments are separated by gel electrophoresis 10. The separated DNA fragments are denaturised using alkali and immobilised on a charged membrane 14 which is placed in a dish 15.

Complementary taggant-labelled oligonucleotides, that is with attached glass bead are produced as described in the examples above or by any other suitable process.

The complementary oligonucleotides are applied to the dish 15 by a pipette 22.

The membrane 14 is left in the dish 15 and incubated further with a minimum volume of hybridisation solution to which the single stranded complementary taggant-labelled oligonucleotides have been added. The membrane 14 and oligonucleotides are incubated for at least six hours at the same temperature that prehybridisation took place.

Following hybridisation the membrane 14 is washed sequentially according to the following schedule:

3 x 10 minutes in 50ml 2 x SSC, 0.1 % SDS at 65°C

3 x 20 minutes in 50ml 0.2 x SSC, 0.1% SDS at 65°C

2 x 10 minutes in 50ml 0.1 x SSC at 37°C.

The membrane is then air-dried and scanned for fluorescence.

**Example of Hybridization Solution for Genomic DNA (30 ml)**

7.5 ml 20x SSC
1.5 ml 100x Denhardt's Solution
3 ml 50% dextran sulfate
1.5 ml 1 M phosphate buffer, pH 6.7
0.3 ml 10% SDS
16.05 ml $H_2O$

Add 150 μl of 10 mg/ml denatured salmon sperm DNA for prehybridization.

**<u>Example 6 - Polystyrene/Antibody Coating</u>**

When proteins, such as antibodies, are bound to the glass beads it is preferred to use a polystyrene linker as described in the method detailed below.

- Take species of glass beads with rare earth dopants.
- Coat glass beads with polystyrene.
- Mix a buffered solution (pH 9.3) comprising an antibody with the polystyrene coated beads.
- Mix the beads in this solution and leave for several hours or overnight at 37°C with gentle shaking.
- Wash two or three times with PBS (phosphate buffered saline) Tween 20 (0.1 %) at room temperature.
- Block beads for 1 hr at 37 °C with 2% BSA (Bovine serum albumin) in PBS to prevent non-specific antibody binding in subsequent steps, then wash as above.
- Wash the beads as above and rinse with distilled water. Beads are ready for testing with the detection system.

Thus for certain embodiments, the glass beads coated with polystyrene and antibodies (as binding agent) are introduced to a sample comprising the antigen (target) also on a polystyrene surface. The sample is then washed and the amount of fluorescence detected from the washed sample is proportional to the amount of glass beads/antibodies bound to the target antigen and therefore indicative of the presence and/or amount of antigen in the sample.

Alternatively a polystyrene surface can be coated with a capture antibody. A mixture of antigens (such as a blood sample) can be introduced, one of which "a first antigen" will bind to the antibody on the polystyrene surface. Then the glass beads with an antigen binding agent are introduced. Said first antigen, functioning as an antibody, will then bind to the antigen attached to the glass beads.

This allows the selection of a specific antigen when the sample comprises a mixture of antigens, for example blood samples comprise many different antigens and it is often only one of these which is under analysis at any one time.

Unbound glass beads can then be washed away and the glass beads bound to the target can be analysed as described above for other examples.

A further option is shown in Figs. 11a - 11d which show antigen 32 immobilised on a polystyrene membrane 34. Unbound antigen 32 is then washed away.

The antigen may be conjugated with BSA (Bovine serum albumin) which is a protein which increases the size of the antigen 32 to aid binding of antibody to the antigen 32.

As shown in Fig. 11b, the remaining sites on the polystyrene membrane 34 are then blocked by the addition of BSA, tween or other suitable agent 38.

As shown in Fig. 11c, free antigen 33 is then introduced along with antibodies 36 in order to produce a competitive binding between the free 33 and immobilised 32 antigens with the antibodies 36. Antibodies 36 which do not bind to free antigen 33, bind to the immobilised antigen 32.

The sample is then washed to wash away any unbound free antigen 33. As shown in Fig. 11d, glass beads 30 with secondary labelled antibodies are added. The secondary labelled antibodies bind to the antibodies 36 (now acting as an antigen). The sample is washed again to separated any unbound glass beads before scanning and detecting the fluorescence as described for other embodiments.

**Fragment analysis**

Certain embodiments of the present invention can be used for DNA fragment analysis, for example, identification purposes. In this example, rare earth labelled glass beads with DNA or oligonucleotides are used as primers in a polymerised chain reaction to produce amplified DNA sequences of varying length that are characteristic for every animal. The procedure is described below.

Glass beads incorporating the rare earth element/dopant are provided with a suitable binding agent, such as a short DNA chain (primer) that is linked to the glass beads. Two different primers are used concurrently; each is synthesised to bind to different members of the two DNA chains being interrogated. One bead-labelled primer binds to each end of the DNA region of interest. Double stranded DNA is then heated up to separate the strands and the primers bind to complementary sequences on each of the strands. Enzymes and single base nucleotides are then introduced to synthesise a DNA chain that is an extension of the bound primer. The extension is complementary to the region of interest. This results in a pair of double stranded DNA chains, one strand of each pair being an original and the other "artificial chain" being made from the enzyme extension, the primer and the glass bead. The artificial strand of each pair is a copy of the original strand of the other pair.

The process is then repeated: the DNA strand pairs are separated by heating and further primers linked to beads and enzymes replicate the strands again. The procedure for conducting a known polymerase chain reaction (PCR) may be followed. This may be repeated, for example thirty times, which allows the original DNA strands to be replicated around one billion times ($2^{30}$). All the strands (apart from the original DNA strands) will have the glass beads attached.

This process can be repeated many times. Included in reaction mix may be different sections of DNA with glass beads with a different spectroscopic signature.

Single strands from each of the DNA strands prepared as detailed above can then be separated by electrophoresis, which is a known technique which separates species primarily according to size by determining the distance each species has moved through the gel after a certain time.

Any unbound glass beads will move quickly through the gel and not be analysed further. The size of the DNA strands can thus be estimated and the strands then scanned to provide a DNA profile for example.

As many as one hundred (or more) strands of similar size can be analysed at one time. Thus, the procedure is far more efficient than performing multiple assays with only three or four strands at a time.

Embodiments of the present invention can also be used in the polymerase chain reaction for applications other than fragment analysis. With such embodiments, labelled beads with attached DNA or oligonucleotides are used as primers in the PCR reaction to produce amplified DNA sequences that are used for varying purposes. For example, they can be used to produce labelled probes two hundred to five hundred base pairs in length that can be used as probes.

The labelled glass bead can also be used in DNA sequencing reactions. According to this application, single nucleotides are labelled with taggant and are used for example in a single-base extension sequencing protocol or the Sanger sequencing method.

The composition can be used with other types of binding assays. For example a whole cell sample can be run on a conventional electrophoretic gel, and blotted to a nitrocellulose membrane as for conventional western blotting. The membrane can then be probed with RE-doped beads bearing the required antibodies against any number of proteins thought to be present in the sample. The membrane can then be developed, and the discrete bands of target proteins in the sample identified by the discrete fluorescence spectra of the RE-ions chosen. For example, antibody 1 can be coupled to RE ion 1, and will bind only to antigen 1 on the blot.
Likewise antigen 2 will be revealed only by the emission spectra of RE ion 2, and so on. Several different targets can thereby be easily identified in the same sample at the same time. In a similar way carrier beads bearing nucleic acid probes can be used in an adapted method of Southern or Northern blotting. The molecular weight of the target can be checked on the blot in order to verify the identification of the target.

Immunoassays have traditionally been performed as discrete tests i.e. one analyte per assay tube. However, embodiments of the present invention allow multianalyte testing in which two or more analytes are measured simultaneously in a single way, with the advantages of work simplification, an increase in test throughput, and possible reduction in the overall cost.
The intrinsically fluorescent lanthanide labels with low background fluorescence, high specific activity, and low non-specific binding are ideal for incorporation into microbead carriers, and the resultant fluorescent signature of the doped bead is highly sensitive, specific, and has a narrow spectrum, making detection of several signatures feasible within a single photometric scan.

For certain embodiments, the detection system can comprise a scanning fluorescence or a scanning confocal fluorescence microscope equipped with a laser source for excitation and fluorescein or phycoerythrin as the label. Different blots (Western, Southern and Northern) can also be used to identify and further characterise the targets found.

## Detection

To distinguish the fluorescence of the doped glass beads attached to the binding agent from other fluorescing species in the sample, the long fluorescence lifetimes of the rare earth (RE) ions were utilised. As the majority of fluorophores have short fluorescence lifetimes in comparison to REs, a pulsed excitation signal would produce from REs a pulsed fluorescence signal of the same frequency producing an alternating current (AC). However, if the pulsed frequency were of a speed where the RE fluorescence did not have time to decay before the next pulse, then a direct current (DC) signal would be produced from REs. This DC signal can be detected without any AC signal interference. Furthermore, as the wavelength of the RE produced fluorescence is very discrete in comparison to other fluorophores, they can be spectrally detected even with multiple-RE doped samples in comparison to molecular fluorescing dyes that have very broad overlapping spectra.

An on/off time (duty cycle) of 20% for a short excitation pulse and long decay time was used. This was altered depending on the lifetime of the dopants used. As the carrier beads are typically under 5μm in diameter, a high power source was required to produce sufficient fluorescence. Therefore small solid state lasers (such as those from Edmund Optics Ltd) were used and selected based on the spectral characterisation of the bulk glass. To increase the excitation and collection efficiency, light guides are used that can bring the source closer to the sample and reduce light scatter.

For detection, small photomultipliers (PMTs) such as the 13mm diameter head on type PMTs from Hammamatsu can be used for high sensitivity in the UV to near IR range. As this system is based on visible excitation and visible emission, this would be suitable for most simple embodiments, but other PMTs can be used for other wavelengths of produced fluorescence. Narrow band filters (10nm bandwidth) can be added to the excitation source and detector to increase the specificity of the detector system and to reduce any background signals. The PMTs are also small enough to fit in a detector head tubes to be as close to the sample as possible with any required lenses or light guides and filters positioned in the tubes.

Instrumentation amplifiers were incorporated in the circuit to amplify the output signals. An electronic low pass active filter was also added before the signal reaches the amplifier to reduce the background AC signal. A cut-off frequency of 2.84Hz was selected to remove any signal with a frequency of greater than 2.84Hz. This effect becomes greater as the frequency increases therefore removes the excitation pulse frequency that is greater than 400Hz.

To increase the detection speed for high throughput analysis a single point detection system was used (as shown in Fig 1). This design can be changed depending on the specific application requirements. The detector head 51 described here can accommodate three different channels (for three different RE dopants) that include three different excitation sources and three detectors. For each channel the excitation and detector are positioned at right angles to each other to increase the fluorescence collection efficiency and to minimise unwanted scattered noise. It also provides an option for the reference detection channel at the centre.

The final signal output can be fed to a pc via a data logger such as a PicoLog ADC11 or a dedicated detection system. This could be used in conjunction with software to verify the beads and their signature present and therefore which antibody and antigen are present in the sample. The signal can be quantified by comparison to standard charts

of known quantities of RE-doped beads.

In use of this arrangement, light is emitted from the emitter, optionally passed through a filter and onto a sample that includes the composition. This light is absorbed by the rare earth dopant, which if it matches the energy levels of the dopant and carrier used causes it to fluoresce. Light emitted from the item is transmitted to the detector. Also the emission from each RE in each carrier decays over a different time period. By virtue of this feature, the time over which an emission occurs for a particular wavelength can be used as part of a signature profile. For a positive test, the light received at the detector should have one or more characteristic features that can be identified.

The spectral emissions of various marker samples have been investigated. As an example, table 4 below shows the emission wavelengths and intensities for various excitation wavelengths for a carrier comprising 3mol% $EuCl_3$ in the borosilicate glass described above. By way of comparison, table 5 shows the corresponding results for the $EuCl_3{:}6H_2O$ dopant, but when in solution. From these tables it can be seen that in glass the strongest excitation is at 395nm, which emits at 615nm and 590.5nm. The corresponding results for the $EuCl_3{:}6H_2O$ in solution shows that the emission wavelengths here are 592.5nm, 618.5nm, 556.5nm, 536nm and 526nm. Hence the spectral response of the marker at 395nm is significantly different from that of the $EuCl_3{:}6H_2O$ in solution. Also in glass, for excitation at a wavelength of 415nm, there is an output of 615nm and 590.5nm. In contrast for the $EuCl_3{:}6H_2O$ in solution there is effectively no fluorescence at this wavelength. Again, this demonstrates that there is significant and measurable difference caused by the incorporation of the $EuCl_3{:}6H_2O$ in the borosilicate carrier.

Table 4

| Excitation Wavelength (nm) | Emission Wavelength (nm) | Fluorescent Intensity |
|---|---|---|
| 395 | 535 | 14.433 |
| 395 | 590.5 | 82.873 |
| 395 | 615 | 285.211 |
| 395 | 654 | 13.332 |
| 415 | 590.5 | 11.469 |
| 415 | 615 | 30.916 |
| 465 | 615 | 175.781 |
| 465 | 590.5 | 38.013 |
| 535 | 615 | 28.495 |

Table 5

| Excitation Wavelength (nm) | Emission Wavelength (nm) | Fluorescent Intensity |
|---|---|---|
| 395 | 526 | 1.746 |
| 395 | 536 | 2.495 |
| 395 | 556.5 | 8.633 |
| 395 | 592.5 | 85.608 |
| 395 | 618.5 | 30.277 |
| 415 | - | - |
| 465 | 594 | 2.288 |
| 465 | 616.5 | 0.793 |
| 465 | 700.5 | 3.915 |
| 535 | 592 | 1.126 |

Because rare earth ions have well defined and relatively narrow, nonoverlapping spectral bands, it is possible to detect the presence of the target molecule using a single discrete pre-determined excitation wavelength and likewise a single discrete pre-determined detection wavelength. For example, for the $EuCl_3$ doped borosilicate glass described

above, the emitter filter could be selected to be 465nm, and the detector filter could be 615nm. Alternatively, a plurality of stimulating wavelengths could be used. To do this, a number of different suitable emitter filters would be selected, and a plurality of corresponding filters. These would be included in the arrangement of Figure 1 to allow the simultaneous measurement of optical response at various different wavelengths. Figure 1 shows a scanning system 50 comprising a photomultiplier 40, a laser head 42, a microscope head 44, a glass sample 46, a shutter 48, a beam chopper 52, a glass slide 54 and a photodiode 56.

A further advantage of the discrete nature of the spectral response of rare earth ions is that a number of species can be combined into the one carrier for a more specific identification signature, for example 3 mole % Eu + 3 mole % Tb, not precluding other rare earths at different percentages and more than two. Because the response of the various different dopants is relatively discrete, detection of these is simplified. The narrow emission bands also facilitate the spectral selection of the molecules, making the detection system simpler that those required for systems containing multiple dyes. A further advantage is that many rare earth ions require excitation at wavelengths conducive to existing laser diode technologies. This makes online excitation not only possible but compact, robust and long lived. Furthermore, incorporating the rare earth dopants into a suitable carrier, and in particular the glass beads described herein, means that the composition in which the invention is embodied is extremely stable under adverse chemical, environmental and physical abrasion conditions.

## Results

The absorption spectra for the europium-doped borosilicate glass are shown in Fig 2 for the whole range and just the visible region. As the sample was a glass, there was a strong absorption in the UV range lower than 300nm which can be ignored for all the samples as this absorption was present for the blank glass absorption shown in Fig 3. As the background absorption from the glass was constant this effect could be removed by taking the second derivative spectrum of each sample.

All the energy level assignments are given in table 6. The peak at 532nm relates to the transition from $^7F_1$-$^5D_1$ whereas all the transitions in the table are from the ground state $^7F_0$.

Table 6 Energy level assignments [1] for $Eu^{3+}$ doped in borosilicate glass

| S'L'J | Excitation Wavelength/nm |
|---|---|
| $^5D_4$ | 362 |
| $^5G_6$ | 377 |
| $^5G_2$ | 381 |
| $^5L_6$ | 393 |
| $^5D_3$ | 413 |
| $^5D_2$ | 464 |
| $^5D_1$ | 526 |
| $^5D_0$ | 579 |

From the absorption spectrum of the blank borosilicate glass there was no absorption in the visible range and therefore no interference. The fluorescence spectrum of the blank glass was analysed to determine whether there was any fluorescence due to the glass and is shown in Fig 4. This spectrum shows a strong broad fluorescence peak ranging from approximately 300-350nm excitation and emission between 350-440nm, which correlates to the absorption spectrum in Fig 3. A closer look at the visible region is also shown in Fig 5.

Figs 4 and 5 do not show any strong fluorescence signal in the visible region in comparison to the UV. Therefore the blank glass does not show any significant fluorescence that would interfere with the rare earth dopants enabling the europium-doped glass to be analysed.

The fluorescence spectra for the 3 mol% europium doped borosilicate glass are shown in Fig 6. These spectra illustrate the sharp characteristic peaks of the rare earths with most of the excitation peaks relating to the absorption spectrum in Fig 2. There was also no signal present from the glass and further backs up the reasoning that the glass would not affect the dopant fluorescence. All the emission transitions for the europium doped glass are shown in table 7

Table 7: Fluorescence peaks their intensity and relative transitions

| Excitation Wavelength/nm | Emission Wavelength/nm | Intensity | Transition |
|---|---|---|---|
| 362 | 591.5 | 40.4522 | $^5D_0$-$^7F_1$ |
| 362 | 616 | 125.969 | $^5D_0$-$^7F_2$ |
| 362 | 654.5 | 11.9613 | $^5D_0$-$^7F_3$ |
| 381 | 592 | 133.643 | $^5D_0$-$^7F_1$ |
| 381 | 615.5 | 465.878 | $^5D_0$-$^7F_2$ |
| 381 | 653.5 | 30.6015 | $^5D_0$-$^7F_3$ |
| 382 | 702.5 | 56.5826 | $^5D_0$-$^7F_4$ |
| 387 | 535 | 40.7422 | $^5D_0$-$^7F_1$ |
| 393 | 591.5 | 218.76 | $^5D_0$-$^7F_1$ |
| 393 | 615.5 | 768.131 | $^5D_0$-$^7F_2$ |
| 393 | 702 | 89.3617 | $^5D_0$-$^7F_4$ |
| 395 | 653 | 43.931 | $^5D_0$-$^7F_3$ |
| 412 | 591 | 59.9294 | $^5D_0$-$^7F_1$ |
| 412 | 615.5 | 166.906 | $^5D_0$-$^7F_2$ |
| 412 | 653.5 | 15.2281 | $^5D_0$-$^7F_3$ |
| 412 | 702.5 | 20.9786 | $^5D_0$-$^7F_4$ |
| 444 | 535.5 | 18.4024 | $^5D_1$-$^7F_1$ |
| 463 | 592 | 121.442 | $^5D_0$-$^7F_1$ |
| 463 | 652 | 33.2972 | $^5D_0$-$^7F_3$ |
| 463 | 702.5 | 42.7858 | $^5D_0$-$^7F_4$ |
| 465 | 535 | 27.624 | $^5D_1$-$^7F_1$ |
| 465 | 615.5 | 479.06 | $^5D_0$-$^7F_2$ |
| 526 | 625.5 | 15.4485 | $^{5D}{}_0$-$^7F_3$ |
| 531 | 590 | 62.7966 | $^5D_0$-$^7F_1$ |
| 531 | 615.5 | 220.965 | $^5D_0$-$^7F_2$ |
| 531 | 702.5 | 24.1966 | $^5D_0$-$^7F_4$ |
| 579 | 616 | 61.5237 | $^5D_0$-$^7F_2$ |
| 579 | 704.5 | 10.4728 | $^5D_0$-$^7F_4$ |
| 580 | 651.5 | 8.48162 | $^5D_0$-$^7F_3$ |

The wavelengths of interest for use with the in-situ detector were 465nm excitation and 615.5nm emission. This peak was useful due to its discrete nature with no interfering peaks around it.

Following the spectral characterisation of the RE doped borosilicate glass, based on discrete excitation & emission wavelengths, europium (Eu) was identified as a suitable dopant. Before using the fluorescent decay time as an identifying feature, it was important to check the fluorescent lifetimes of the doped RE ions inside the glass.

In order to test the lifetimes, time resolved fluorescence studies of the ions in the borosilicate glass were carried out. The experimental set-up which includes the Laser Induced Scanning Fluorescence Microscope (LISFM) is shown in Fig 1. The microscope focused the laser light on to the RE doped glass sample and collected the fluorescence. Short laser pulses of appropriate wavelength, generated from a continuous wave (CW) Ar-ion laser by a mechanical chopper, were used to excite the fluorescence and the corresponding temporal fluorescence intensity variations were detected using a highly sensitive photodetector (Photomultiplier tube or photodiode). A set of filters were optionally placed in front of the detector to filter-out unwanted wavelengths. As a reference, the laser pulses were monitored using a photodiode

with the help of a partially reflecting glass plate (microscopic glass slide). A Tektronix TDS 380 digital real-time oscilloscope was used to display and record the signals.

Spectral characteristics of Eu doped samples have shown a strong absorption peak around 465nm and corresponding emission peak around 614nm. Hence an Ar-ion laser wavelength at 465nm was selected for the excitation and an interference filter with transmission peak at 620nm (Full Width at Half Maximum, FWHM = 10nm) was chosen as the fluorescence filter. The transmitted intensity was detected using a PMT and was displayed/recorded using the oscilloscope. A typical laser pulse is shown in Fig 8 and has a pulse width of almost 500 microseconds (FWHM). The corresponding fluorescent pulse from 3 mol% Eu doped in borosilicate glass is shown in Fig 9. As can be observed from the figure, the fluorescence pulse is much longer than the pump pulse (nearly 7millisecond base width) and has a FWHM of - 2millisecond.

The secondary label of the FITC conjugated to the antibody can be used to quantify the amount of antigen present, by comparison with a known amount in standard graphs.

While only a few rare earth ions have been specifically described, it will be appreciated that there is a wide range of fluorescent rare earth ions that could be used. The permutations available are therefore greatly enhanced. Excitation and emission peaks for some RE ions are shown in table 8 as follows:

Table 8

| RE Ions | Max Excitation peak (nm) | Max Emission Peak (nm) |
|---|---|---|
| $Eu^{3+}$ | 395 | 614 |
| $Tb^{3+}$ | 380 | 545 |
| $Dy^{3+}$ | 350 | 575 |

In addition, whilst some rare earth ions emit in UV and IR ranges, it is preferred that both the excitation radiation and the emitted radiation are within the visible range, that is, within a wavelength range that is visible to the unaided human eye. Accordingly, the above description of a specific embodiment is made by way of example only and not for the purposes of limitation.

## References

1. Carnall, W.T., Fields, P.R. and Rajnak, K., Electronic energy levels of the trivalent lanthanide aquo ions. IV. Eu3+, The Journal of Chemical Physics, 1968, 49(10), 4450-4455

2. Mullin, J.W., Particle size analysis with micro sieves using ultrasonic vibrations, Chemistry and Industry, 1971, 1435-1436

3. Rawle, A., Basic principles of particle size analysis, Accessed 26/05/03,http://www.malvern.co.uk/malvern/kbase.nsf/0/5E3F5A148D336B0480256 BF2006E2195/$file/Basic principles of particle size analysis MRK034-low res.pdf.

4. Kumar, A, Larsson, O, Parodi, D and Liang Z (2000). Silanised nucleic acids: a general platform for DNA immobilization. Nucleic Acids Research Methods Online, 28, 14, e71


## Claims

1. An optically detectable analytical composition comprising glass particles of less than 20μm, the said particles consisting of a glass carrier incorporating a rare earth dopant, and, bound to the carrier, at least one of a biological binding agent selected from biomolecules, and macromolecules, or a chemical linker suitable to bind to a biological binding agent, said linker providing a means of modifying the surface of the glass carrier to render it receptive to attachment of a biomolecule or macromolecule, ***characterised in that*** the glass is a borosilicate glass.

2. A composition as claimed in claim 1, wherein the biological binding agent is selected from oligonucleotides, nucleic acids, alleles, genes, enzymes, antibodies, antigens and cellular species.

3. A composition as claimed in claim 1, wherein the glass particles are of less than 5μm.

4. A composition as claimed in claim 1, wherein the chemical linker comprises one of a silane or polystyrene.

5. A composition as claimed in claim 1, wherein the chemical linker comprises any one from the group consisting of a thiol group, an activated carboxylic group, an iodo-acetamide group and a maleimide group.

6. A composition as claimed in any preceding claim, wherein the composition comprises a plurality of different rare earth dopants.

7. A composition as claimed in claim 6, wherein the rare earth dopants are selected from Europium, Dysprosium, and Terbium.

8. A composition as claimed in claim 6, wherein the different rare earth dopants each have different concentrations levels, such that the intensities of the pre-selected wavelength emissions are different.

9. A method of analysing a target in a sample, the method comprising:

(a) providing an analysis composition as in claim 1, wherein a biological binding agent conjugated to a particle is adapted or chosen to bind to the target;
(b) exposing the sample and the biological binding agent to each other;
(c) separating (i) the biological binding agent which has bound to any target from (ii) the biological binding agent which has not bound to any target;
(d) and, to produce emissions from the rare earth element, illuminating at least one of (i) the biological binding agent which has bound to any target and (ii) the biological binding agent which has not bound to any target;
(e) detecting any emission from the sample illuminated in step (e); and,
(f) relating the detected emission to any feature of the target including its presence or absence in the sample.

10. A method as claimed in claim 9, wherein said feature is the presence or absence of the target in the sample.

11. A method as claimed in either one of claims 9 or 10, wherein the amount of any target is determined by the detected emission.

12. A method as claimed in claim 9, wherein said feature is the size of the target molecules.

13. A method as claimed in any one of claims 9 to 10, wherein the conjugated biological binding agent is such as to cause the composition to emit light that is of a wavelength in the visible region of the electromagnetic spectrum between 390nm and 700nm.

14. A method as claimed in any one of claims 9 to 13, wherein the biological binding agent comprises a nucleotide.

15. A method as claimed in claim 14, including the step of performing a hybridisation.

16. A method as claimed in claim 13 or claim 14, used to conduct fragment analysis of nucleic acids, such as DNA.

17. A method as claimed in any one of claims 9 to 13, wherein one of the biological binding agent and target is an antibody and the other of the biological binding agent and target is an antigen.

18. A method as claimed in any one of claims 9 to 13, wherein the biological binding agent is one of a protein and a cellular species and the target is the other of the protein and cellular species.

19. A method as claimed in any one of claims 9 to 18, wherein the carrier comprises glass beads and silanisation of the glass beads is performed before the biological molecule is conjugated thereto.

20. A method as claimed in any one of claims 9 to 19, wherein more than one type of target molecule is bound.

**Patentansprüche**

1. Eine optisch nachweisbare analytische Zusammensetzung, die Folgendes beinhaltet: Glaspartikel von weniger als 20 μm, wobei die Partikel aus einem Glasträger, der ein Seltenerd-Dotiermittel inkorporiert, bestehen, und, an den Träger gebunden, mindestens eines aus einem biologischen Bindemittel, das aus Biomolekülen und Makromolekülen ausgewählt ist, oder einem chemischen Linker, der geeignet ist, um an ein biologisches Bindemittel zu binden, wobei der Linker ein Mittel zum Modifizieren der Oberfläche des Glasträgers bereitstellt, um sie für die Anlagerung eines Biomoleküls oder Makromoleküls empfänglich zu machen, **dadurch gekennzeichnet, dass** das Glas ein Borsilicatglas ist.

2. Zusammensetzung gemäß Anspruch 1, wobei das biologische Bindemittel aus Oligonukleotiden, Nukleinsäuren, Allelen, Genen, Enzymen, Antikörpern, Antigenen und Zelltypen ausgewählt ist.

3. Zusammensetzung gemäß Anspruch 1, wobei die Glaspartikel kleiner als 5 μm sind.

4. Zusammensetzung gemäß Anspruch 1, wobei der chemische Linker eines aus Silan oder Polystyrol beinhaltet.

5. Zusammensetzung gemäß Anspruch 1, wobei der chemische Linker eines aus der Gruppe, bestehend aus einer Thiolgruppe, einer aktivierten Carboxylgruppe, einer lodacetamidgruppe und einer Maleimidgruppe, beinhaltet.

6. Zusammensetzung gemäß einem der vorhergehenden Ansprüche, wobei die Zusammensetzung eine Vielzahl von unterschiedlichen Seltenerd-Dotiermitteln beinhaltet.

7. Zusammensetzung gemäß Anspruch 6, wobei die Seltenerd-Dotiermittel aus Europium, Dysprosium und Terbium ausgewählt sind.

8. Zusammensetzung gemäß Anspruch 6, wobei die unterschiedlichen Seltenerd-Dotiermittel jeweils unterschiedliche Konzentrationsgrade aufweisen, so dass die Intensitäten der Emissionen bei zuvor ausgewählten Wellenlängen unterschiedlich sind.

9. Ein Verfahren zum Analysieren eines Ziels in einer Probe, wobei das Verfahren Folgendes beinhaltet:

   (a) Bereitstellen einer Analysezusammensetzung gemäß Anspruch 1, wobei ein biologisches Bindemittel, das mit einem Partikel konjugiert ist, angepasst oder gewählt ist, um an das Ziel zu binden;
   (b) Einander-Aussetzen der Probe und des biologischen Bindemittels;
   (c) Trennen (i) des biologischen Bindemittels, das an ein beliebiges Ziel gebunden hat, von (ii) dem biologischen Bindemittel, das nicht an ein beliebiges Ziel gebunden hat;
   (d) und Beleuchten von mindestens einem von (i) dem biologischen Bindemittel, das an ein beliebiges Ziel gebunden hat, und (ii) dem biologischen Bindemittel, das nicht an ein beliebiges Ziel gebunden hat, um Emissionen von dem Seltenerdmetall zu produzieren;
   (e) Nachweisen von jeglicher Emission aus der in Schritt (d) beleuchteten Probe und
   (f) In-Beziehung-Bringen der nachgewiesenen Emission mit einem beliebigen Merkmal des Ziels einschließlich seines Vorhandenseins in oder seiner Abwesenheit von der Probe.

10. Verfahren gemäß Anspruch 9, wobei das Merkmal das Vorhandensein oder die Abwesenheit des Ziels in der Probe ist.

11. Verfahren gemäß einem der Ansprüche 9 oder 10, wobei die Menge eines beliebigen Ziels durch die nachgewiesene Emission bestimmt wird.

12. Verfahren gemäß Anspruch 9, wobei das Merkmal die Größe der Zielmoleküle ist.

13. Verfahren gemäß einem der Ansprüche 9 bis 10, wobei das konjugierte biologische Bindemittel derart ist, dass es die Zusammensetzung dazu bringt, Licht zu emittieren, das von einer Wellenlänge in dem sichtbaren Bereich des elektromagnetischen Spektrums zwischen 390 nm und 700 nm ist.

14. Verfahren gemäß einem der Ansprüche 9 bis 13, wobei das biologische Bindemittel ein Nukleotid beinhaltet.

**15.** Verfahren gemäß Anspruch 14, das den Schritt des Durchführens einer Hybridisierung umfasst.

**16.** Verfahren gemäß Anspruch 13 oder Anspruch 14, das verwendet wird, um eine Fragmentanalyse von Nukleinsäuren wie etwa DNA auszuführen.

**17.** Verfahren gemäß einem der Ansprüche 9 bis 13, wobei eines von dem biologischen Bindemittel und dem Ziel ein Antikörper ist und das andere von dem biologischen Bindemittel und dem Ziel ein Antigen ist.

**18.** Verfahren gemäß einem der Ansprüche 9 bis 13, wobei das biologische Bindemittel eines von einem Protein und einem Zelltyp ist und das Ziel das andere von dem Protein und dem Zelltyp ist.

**19.** Verfahren gemäß einem der Ansprüche 9 bis 18, wobei der Träger Glasperlen beinhaltet und eine Silanisierung der Glasperlen durchgeführt wird, bevor das biologische Molekül damit konjugiert wird.

**20.** Verfahren gemäß einem der Ansprüche 9 bis 19, wobei mehr als eine Art von Zielmolekül gebunden wird.

**Revendications**

**1.** Une composition analytique pouvant être détectée optiquement comprenant des particules de verre de moins de 20 $\mu$m, lesdites particules consistant en un support en verre incorporant un dopant aux terres rares, et, lié au support, au moins soit un agent de liaison biologique sélectionné parmi des biomolécules, et des macromolécules, soit un coupleur chimique adapté pour se lier à un agent de liaison biologique, ledit coupleur apportant un moyen de modifier la surface du support en verre pour la rendre réceptive à l'attachement d'une biomolécule ou d'une macromolécule, **caractérisée en ce que** le verre est un verre borosilicaté.

**2.** Une composition telle que revendiquée dans la revendication 1, où l'agent de liaison biologique est sélectionné parmi des oligonucléotides, des acides nucléiques, des allèles, des gènes, des enzymes, des anticorps, des antigènes et des espèces cellulaires.

**3.** Une composition telle que revendiquée dans la revendication 1, où les particules de verre font moins de 5 $\mu$m.

**4.** Une composition telle que revendiquée dans la revendication 1, où le coupleur chimique comprend soit un silane, soit un polystyrène.

**5.** Une composition telle que revendiquée dans la revendication 1, où le coupleur chimique comprend un groupement quelconque du groupe constitué d'un groupement thiol, un groupement carboxylique activé, un groupement iodoacétamide et un groupement maléimide.

**6.** Une composition telle que revendiquée dans n'importe quelle revendication précédente, où la composition comprend une pluralité de dopants aux terres rares différents.

**7.** Une composition telle que revendiquée dans la revendication 6, où les dopants aux terres rares sont sélectionnés parmi l'europium, le dysprosium et le terbium.

**8.** Une composition telle que revendiquée dans la revendication 6, où les différents dopants aux terres rares ont chacun des niveaux de concentrations différents, de sorte que les intensités des émissions à des longueurs d'ondes présélectionnées sont différentes.

**9.** Une méthode d'analyse d'une cible dans un échantillon, la méthode comprenant :

(a) fournir une composition d'analyse telle que dans la revendication 1, où un agent de liaison biologique conjugué à une particule est adapté ou choisi pour se lier à la cible ;
(b) exposer l'échantillon et l'agent de liaison biologique l'un à l'autre ;
(c) séparer (i) l'agent de liaison biologique qui s'est lié à une cible quelconque de (ii) l'agent de liaison biologique qui ne s'est lié à aucune cible ;
(d) et, afin de produire des émissions à partir du métal du groupe des terres rares, illuminer au moins soit (i) l'agent de liaison biologique qui s'est lié à une cible quelconque, soit (ii) l'agent de liaison biologique qui ne

s'est lié à aucune cible ;
(e) détecter toute émission provenant de l'échantillon illuminé au cours de l'étape (d) ; et,
(f) rapporter l'émission détectée à toute caractéristique de la cible y compris sa présence ou son absence dans l'échantillon.

**10.** Une méthode telle que revendiquée dans la revendication 9, où ladite caractéristique est la présence ou l'absence de la cible dans l'échantillon.

**11.** Une méthode telle que revendiquée dans l'une ou l'autre des revendications 9 et 10, où la quantité de toute cible est déterminée par l'émission détectée.

**12.** Une méthode telle que revendiquée dans la revendication 9, où ladite caractéristique est la taille des molécules de la cible.

**13.** Une méthode telle que revendiquée dans une quelconque des revendications 9 à 10, où l'agent de liaison biologique conjugué est tel à amener la composition à émettre de la lumière qui est d'une longueur d'onde dans le domaine visible du spectre électromagnétique comprise entre 390 nm et 700 nm.

**14.** Une méthode telle que revendiquée dans une quelconque des revendications 9 à 13, où l'agent de liaison biologique comprend un nucléotide.

**15.** Une méthode telle que revendiquée dans la revendication 14, incluant l'étape consistant à opérer une hybridation.

**16.** Une méthode telle que revendiquée dans la revendication 13 ou la revendication 14, utilisée pour réaliser une analyse de fragments d'acides nucléiques, tels que l'ADN.

**17.** Une méthode telle que revendiquée dans une quelconque des revendications 9 à 13, où entre l'agent de liaison biologique et la cible, l'un est un anticorps, et l'autre est un antigène.

**18.** Une méthode telle que revendiquée dans une quelconque des revendications 9 à 13, où l'agent de liaison biologique est un élément parmi une protéine et une espèce cellulaire et la cible est l'autre élément parmi la protéine et l'espèce cellulaire.

**19.** Une méthode telle que revendiquée dans une quelconque des revendications 9 à 18, où le support comprend des billes de verre et les billes de verre sont silanisées avant que la molécule biologique soit conjuguée à celles-ci.

**20.** Une méthode telle que revendiquée dans une quelconque des revendications 9 à 19, où plus d'un type de molécule de la cible est lié.

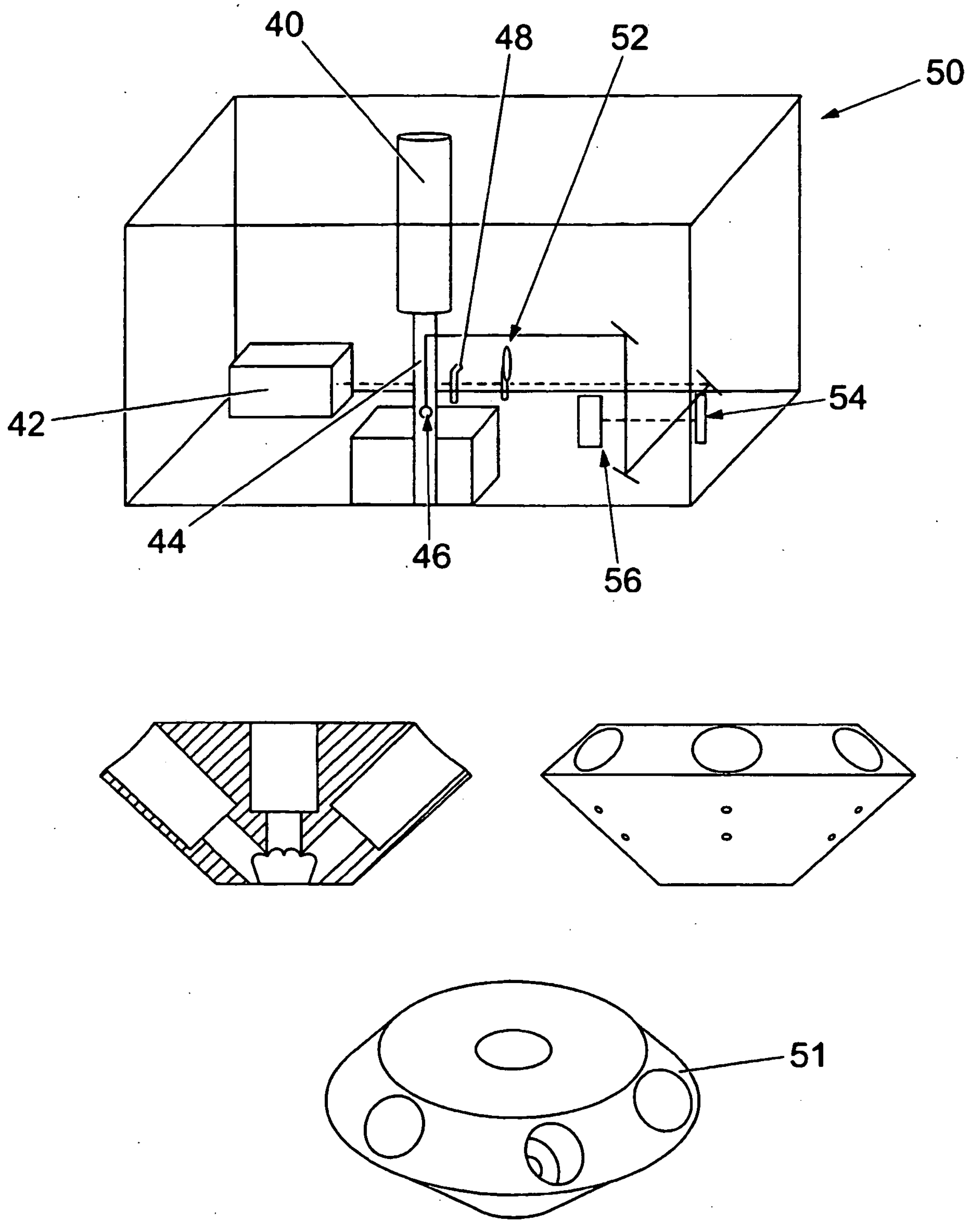


*Fig. 1*

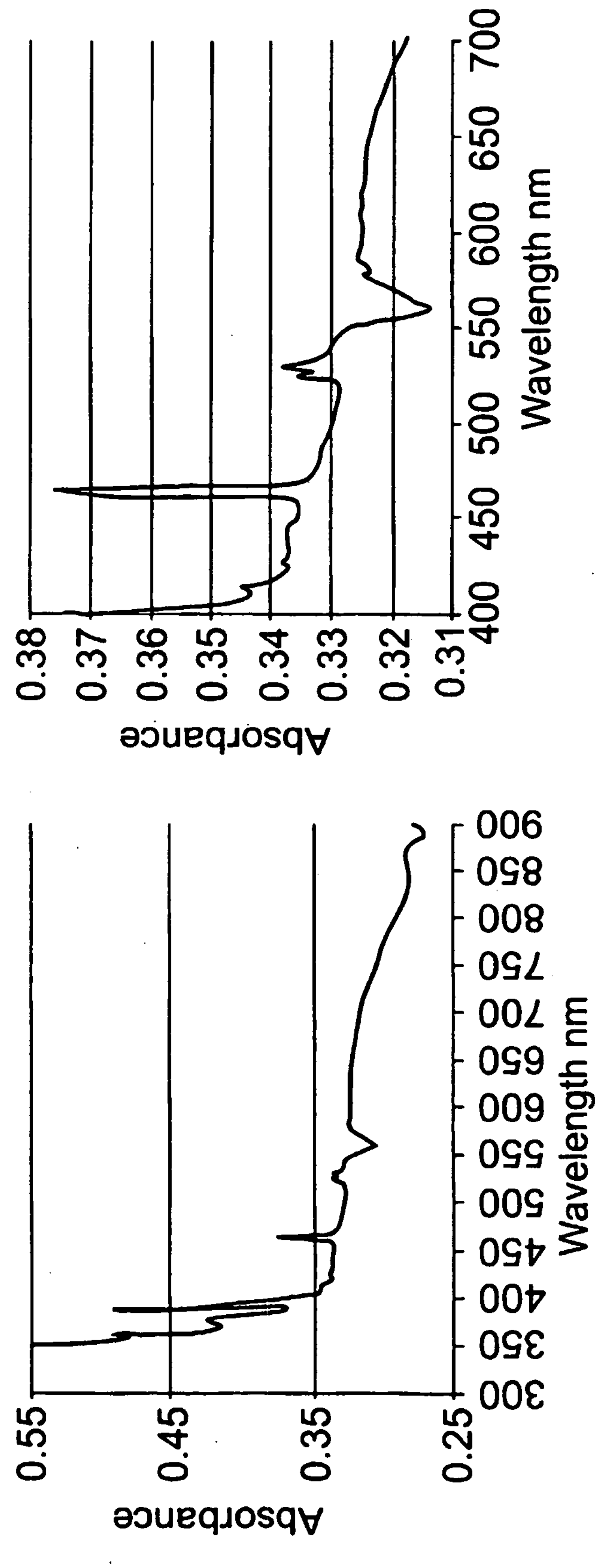
Absorbance
0.38
0.37
0.36
0.35
0.34
0.33
0.32
0.31
400
450
500
550
600
650
700
Wavelength nm
Absorbance
0.55
0.45
0.35
0.25
300
350
400
450
500
550
600
650
700
750
800
850
900
Wavelength nm

*Fig. 2*

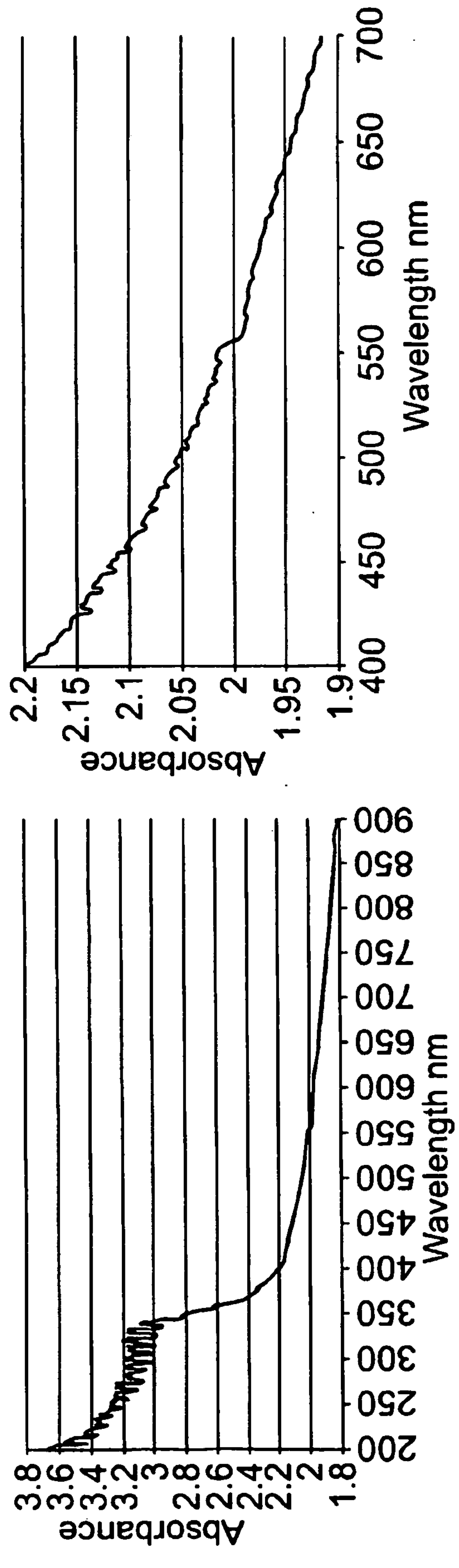
Absorbance
2.2
2.15
2.1
2.05
2
1.95
1.9
400
450
500
550
600
650
700
Wavelength nm
Absorbance
3.8
3.6
3.4
3.2
3
2.8
2.6
2.4
2.2
2
1.8
200
250
300
350
400
450
500
550
600
650
700
750
800
850
900
Wavelength nm

*Fig. 3*

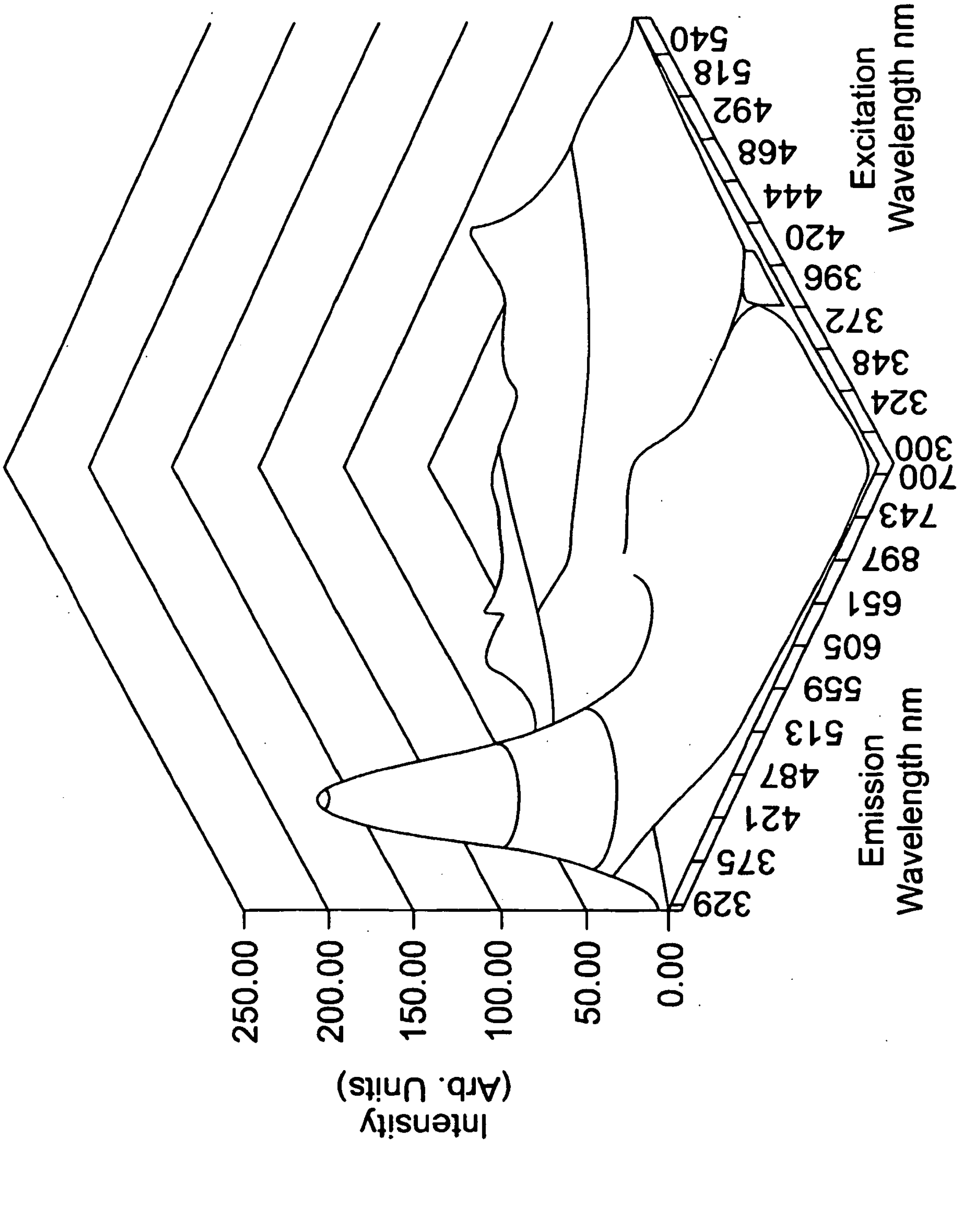
Intensity
(Arb. Units)
250.00
200.00
150.00
100.00
50.00
0.00
Emission
Wavelength nm
329
375
421
467
513
559
605
651
697
743
700
300
324
348
372
396
420
444
468
492
518
540
Excitation
Wavelength nm

*Fig. 4*

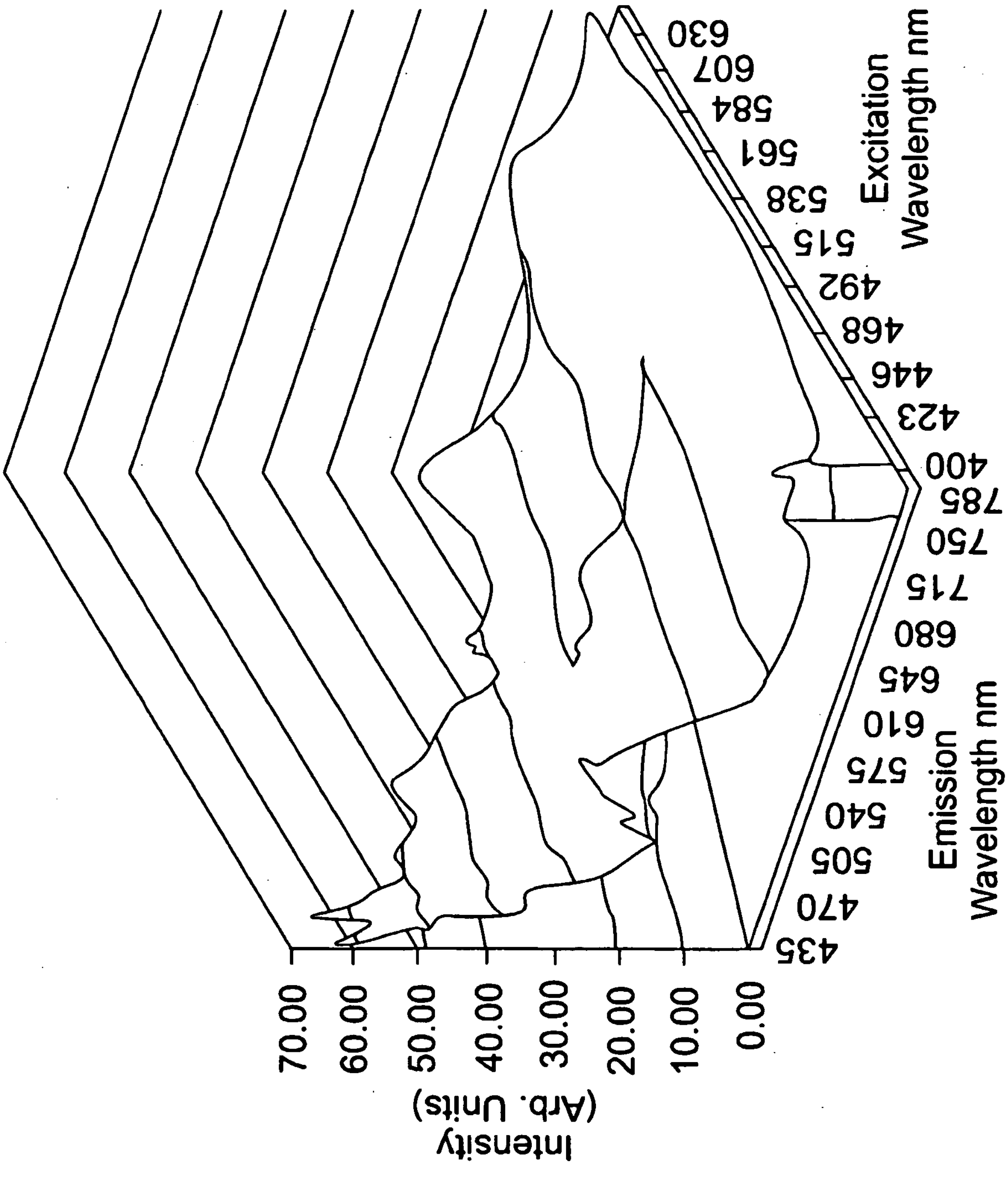
Intensity
(Arb. Units)
70.00
60.00
50.00
40.00
30.00
20.00
10.00
0.00
435
470
505
540
575
610
645
680
715
750
785
Emission
Wavelength nm
400
423
446
468
492
515
538
561
584
607
630
Excitation
Wavelength nm

*Fig. 5*

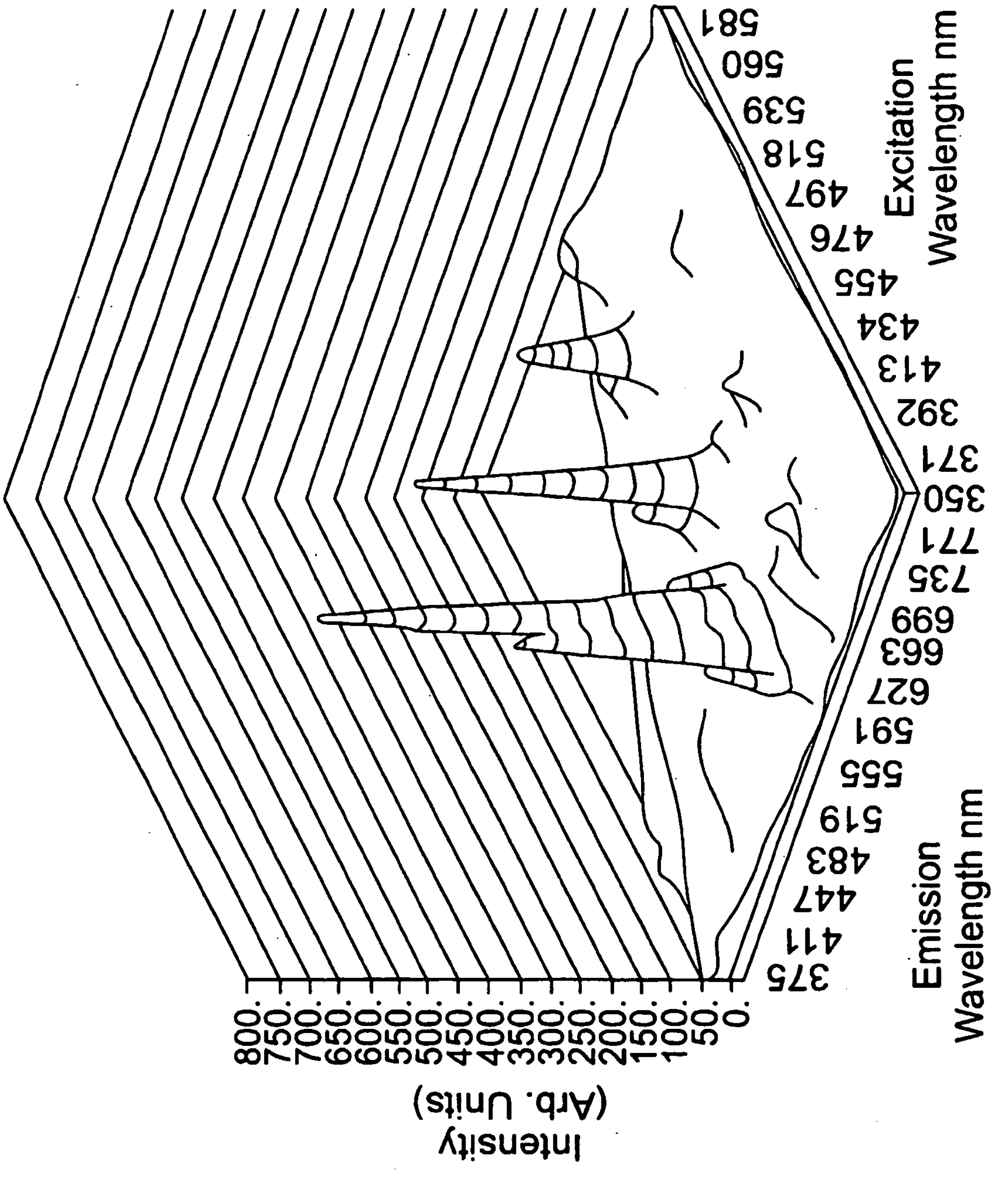
Intensity (Arb. Units)
800. 750. 700. 650. 600. 550. 500. 450. 400. 350. 300. 250. 200. 150. 100. 50. 0.
375 411 447 483 519 555 591 627 663 699 735 771
Emission Wavelength nm
350 371 392 413 434 455 476 497 518 539 560 581
Excitation Wavelength nm

*Fig. 6a*

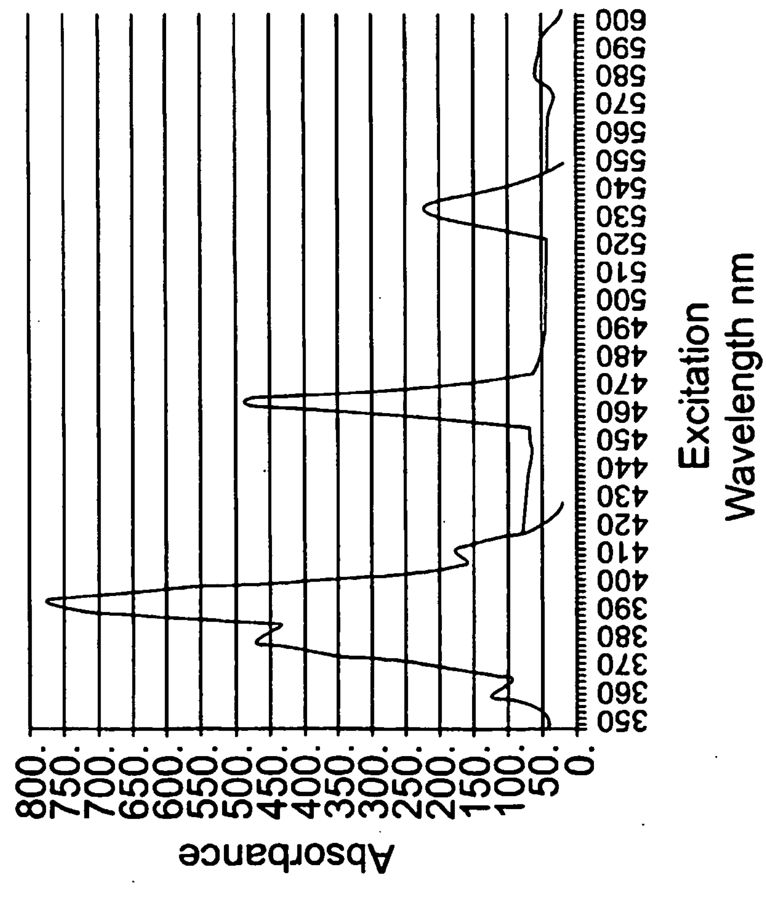
Absorbance
800.
750.
700.
650.
600.
550.
500.
450.
400.
350.
300.
250.
200.
150.
100.
50.
0.
350 360 370 380 390 400 410 420 430 440 450 460 470 480 490 500 510 520 530 540 550 560 570 580 590 600
Excitation
Wavelength nm

Fig. 6b

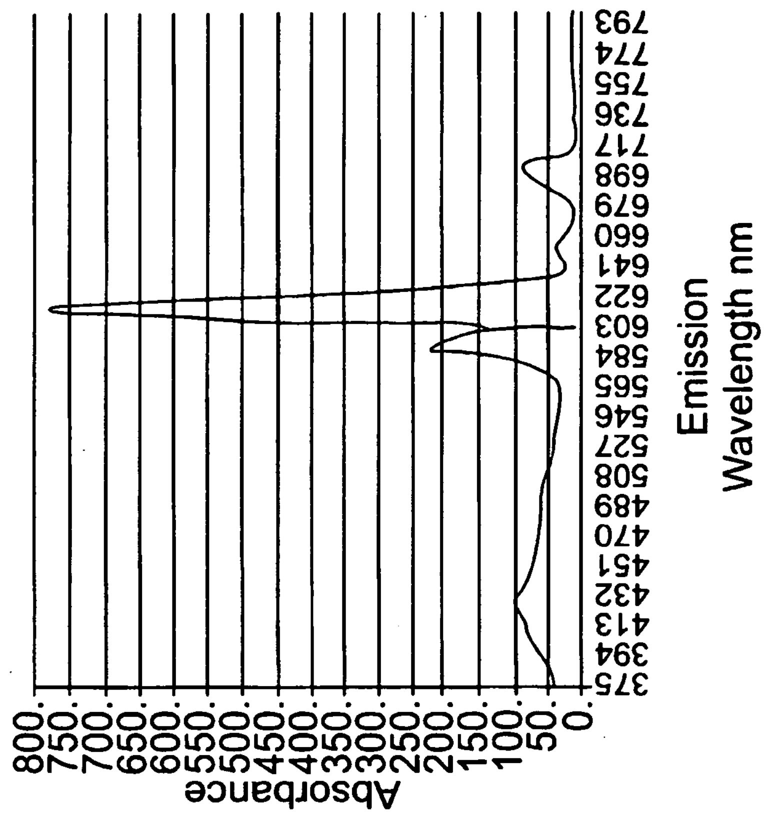
Absorbance
800.
750.
700.
650.
600.
550.
500.
450.
400.
350.
300.
250.
200.
150.
100.
50.
0.
375 394 413 432 451 470 489 508 527 546 565 584 603 622 641 660 679 698 717 736 755 774 793
Emission
Wavelength nm

Fig. 6c

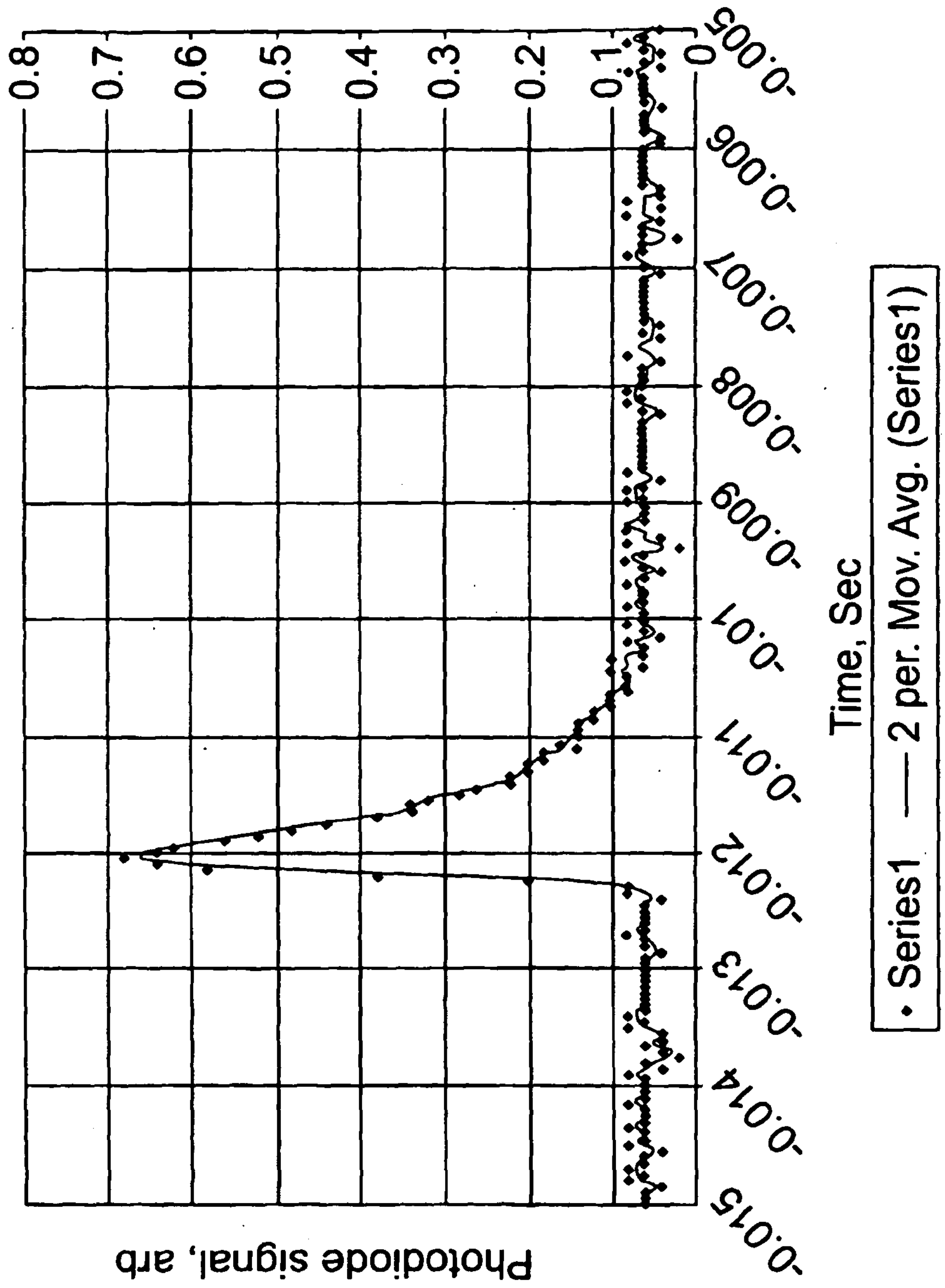
Photodiode signal, arb
0
0.1
0.2
0.3
0.4
0.5
0.6
0.7
0.8
-0.005
-0.006
-0.007
-0.008
-0.009
-0.01
-0.011
-0.012
-0.013
-0.014
-0.015
Time, Sec
• Series1 —— 2 per. Mov. Avg. (Series1)

*Fig. 7*

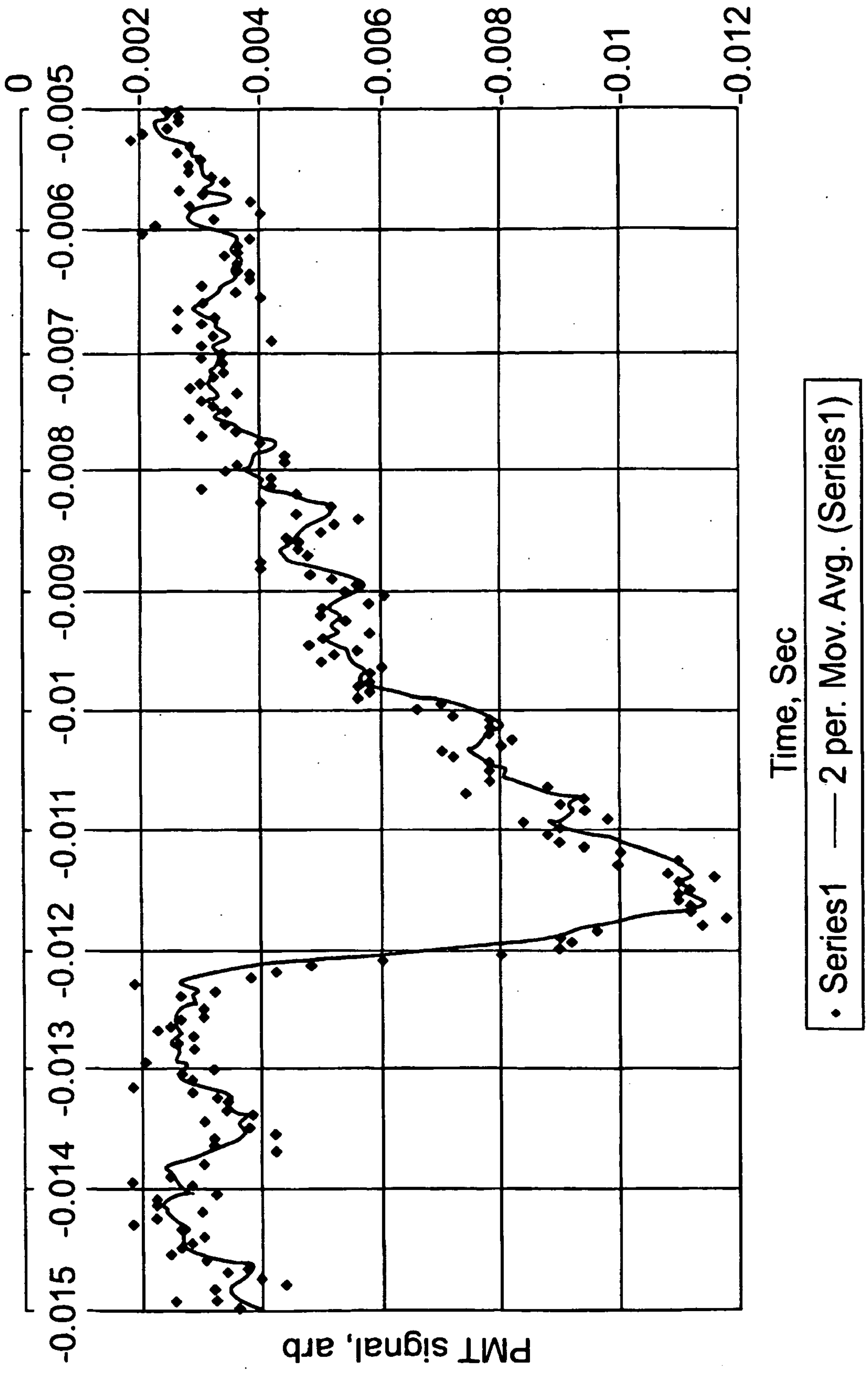
0
-0.002
-0.004
-0.006
-0.008
-0.01
-0.012
-0.015
-0.014
-0.013
-0.012
-0.011
-0.01
-0.009
-0.008
-0.007
-0.006
-0.005
PMT signal, arb
Time, Sec
• Series1 —— 2 per. Mov. Avg. (Series1)

*Fig. 8*

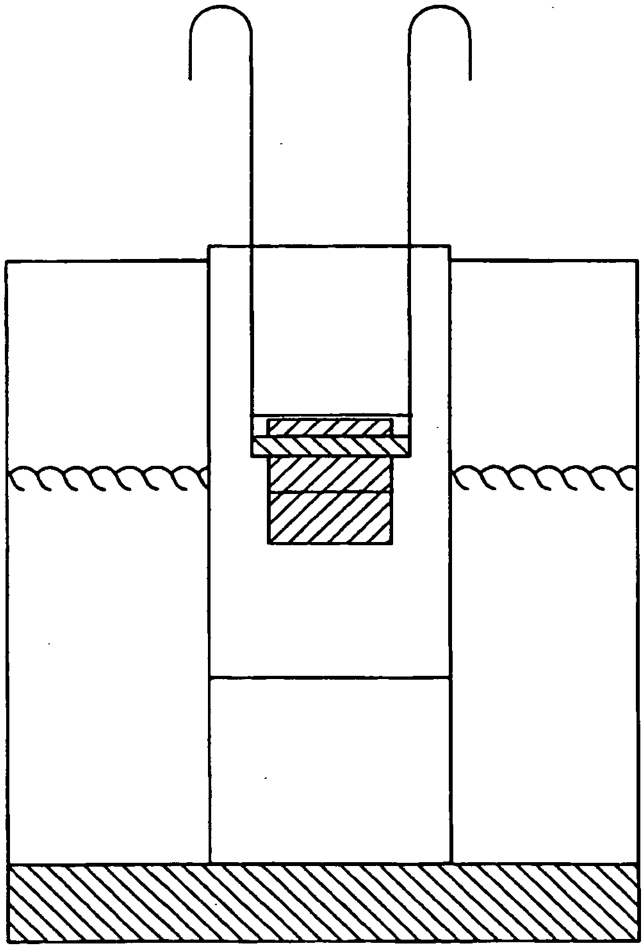

Fig. 9

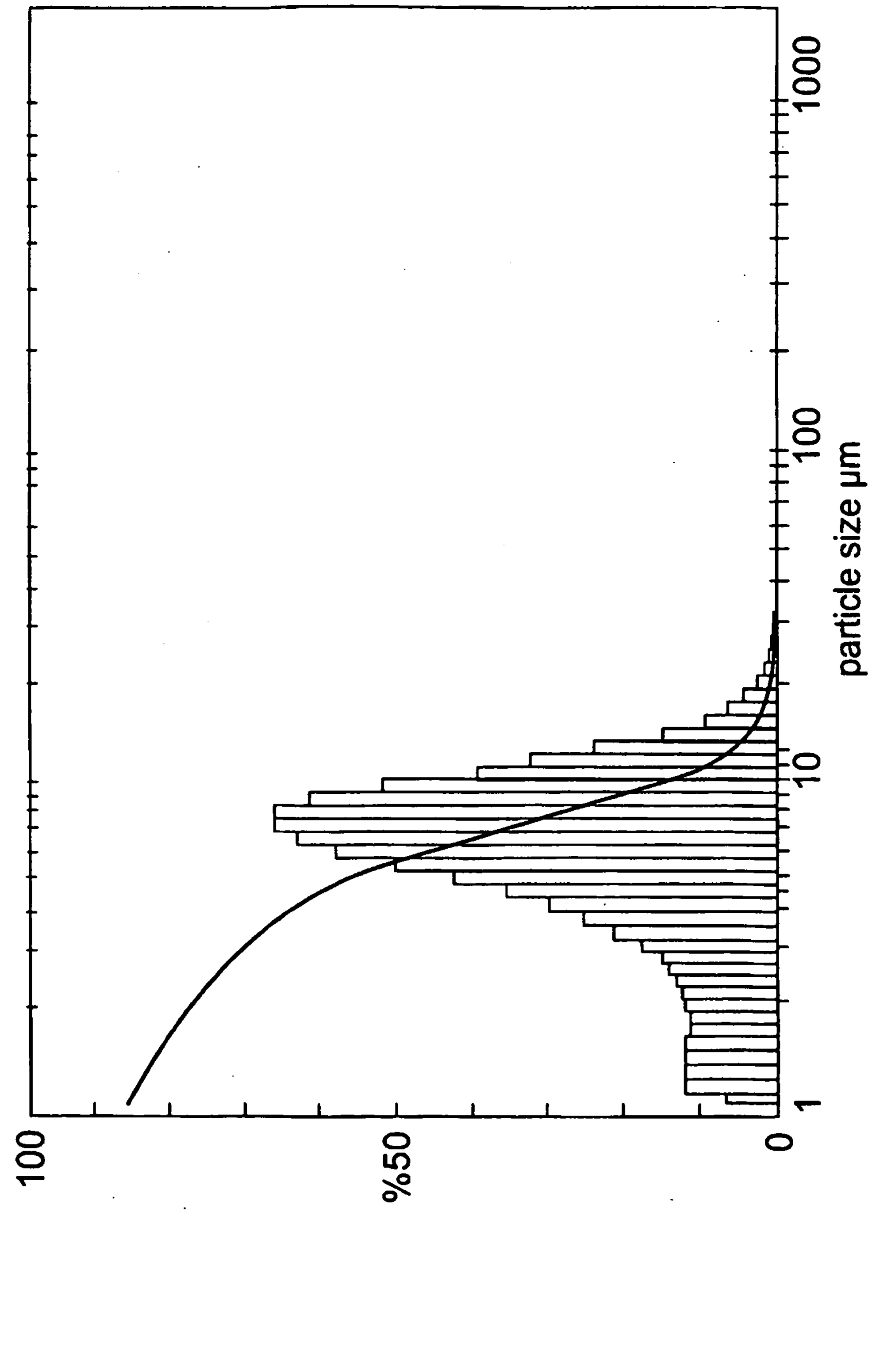


*Fig. 10*

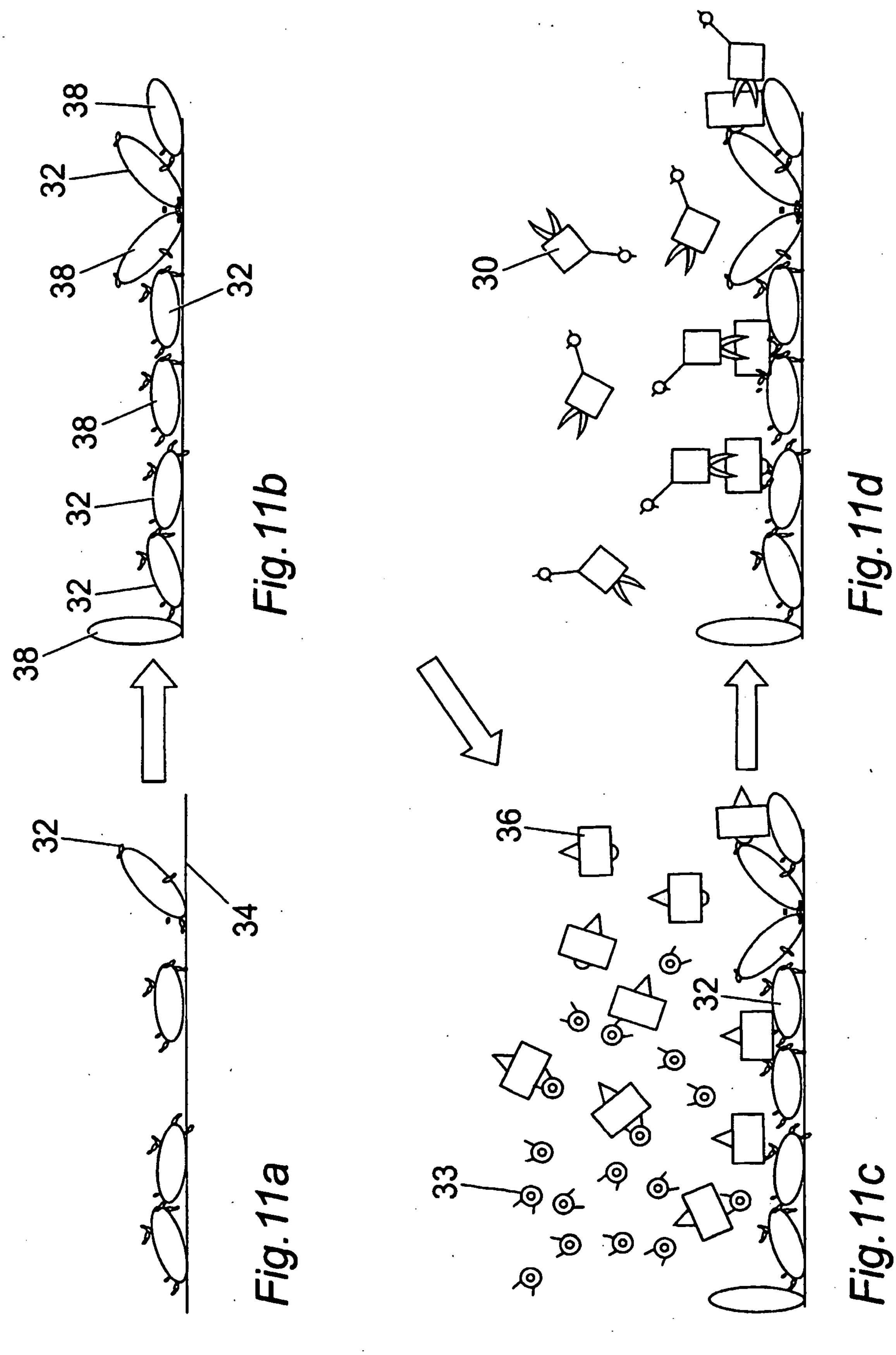
Fig.11a
32
34
Fig.11b
38
32
Fig.11c
33
36
32
Fig.11d
30

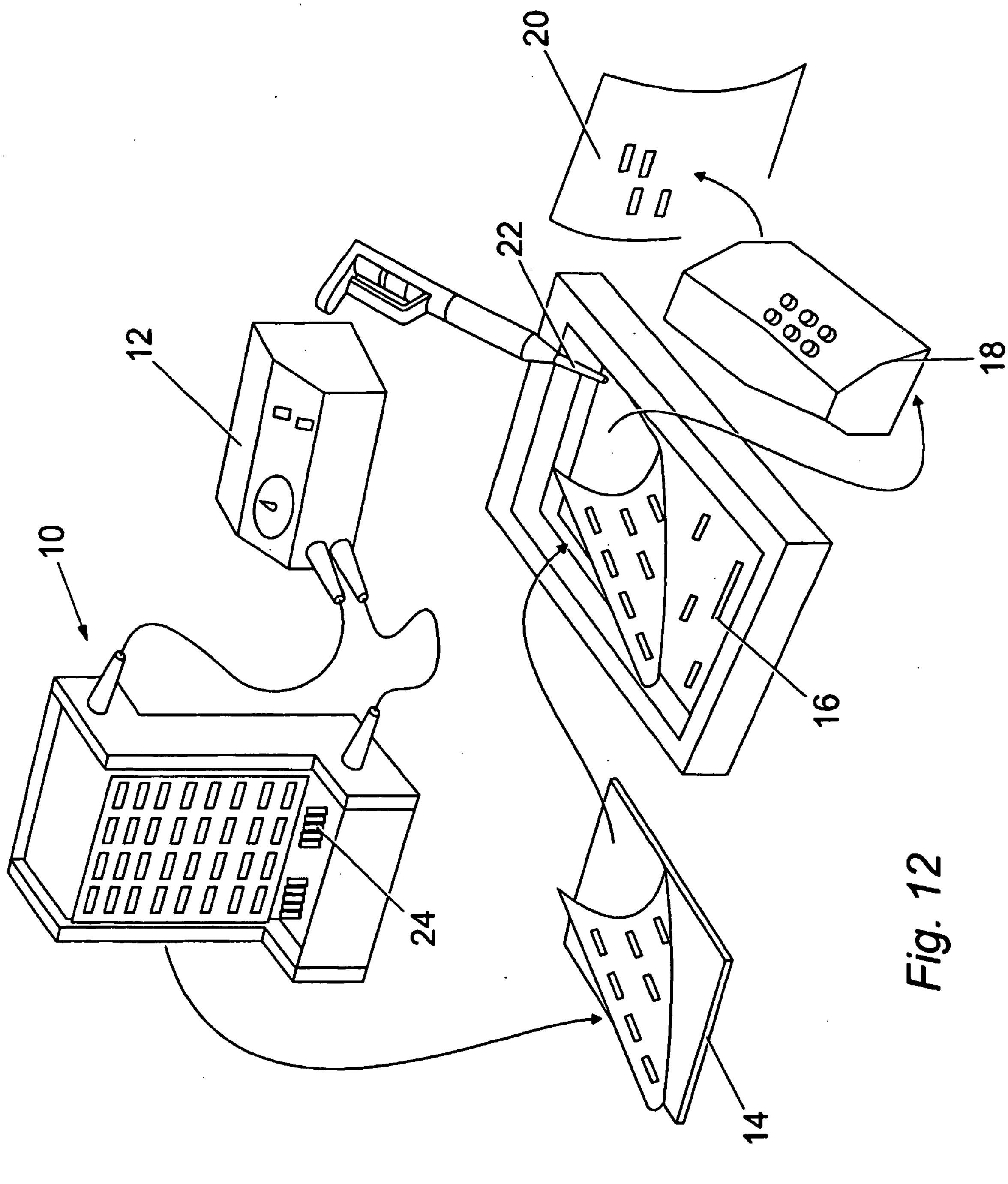


Fig. 12

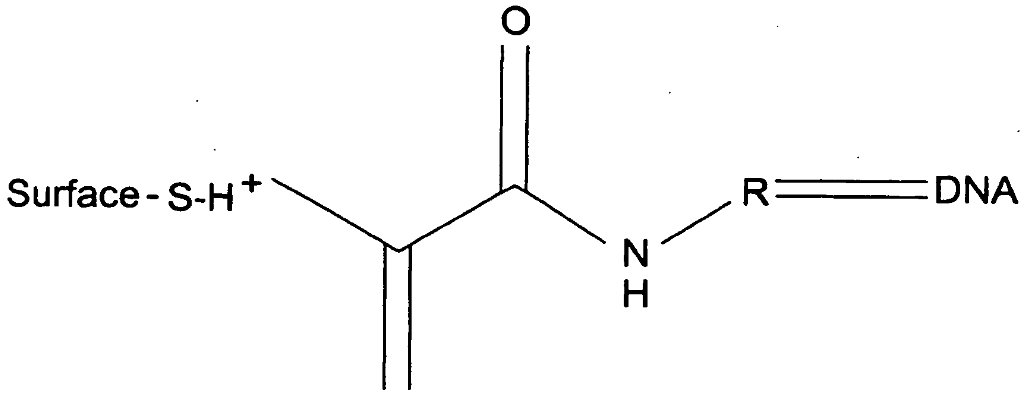

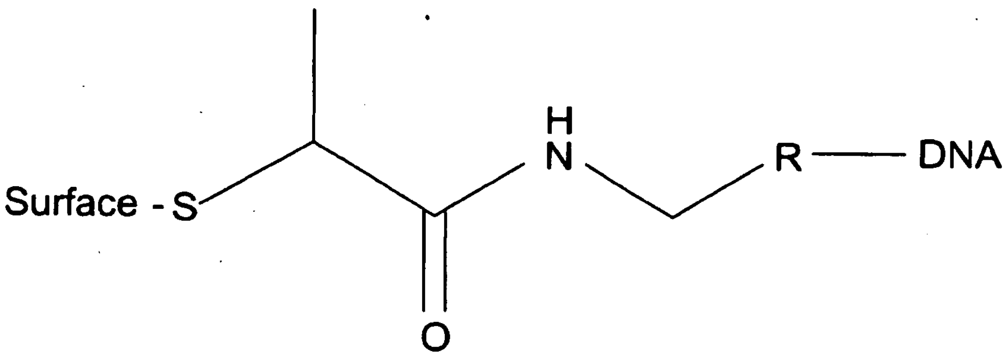

*Fig. 13*

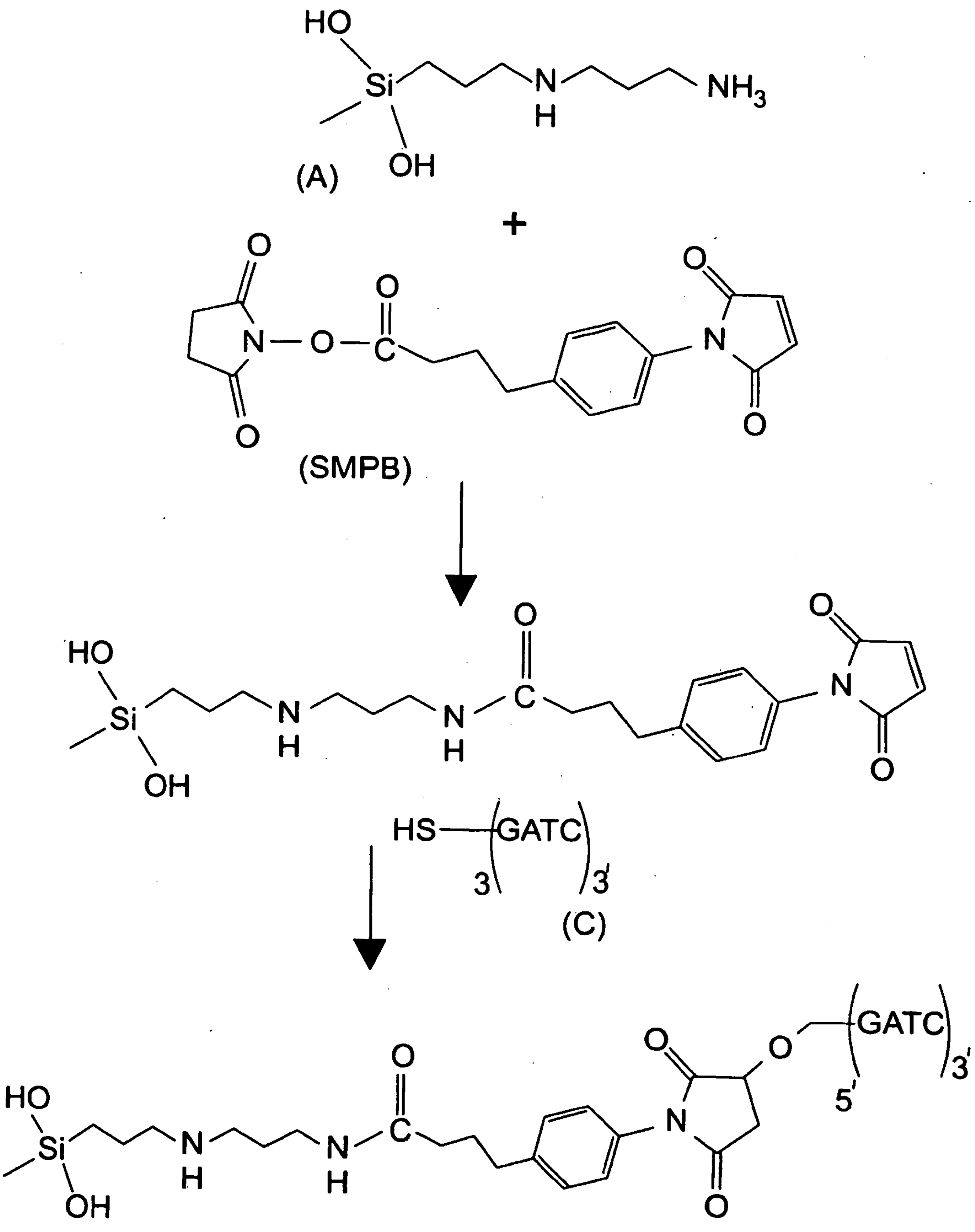

(A)

(SMPB)

(C)

*Fig. 14*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20040171076 A1, Matthew J. Dejneka


### Non-patent literature cited in the description


- **Härmä et al.** *Clin Chem,* vol. 47 (3), 561
- **Matthew J. Dejneka.** *Proceedings of the National Academy of Sciences of the United States of America,* 21 January 2003, vol. 100 (2), 389-393
- **Carnall, W.T. ; Fields, P.R. ; Rajnak, K.** Electronic energy levels of the trivalent lanthanide aquo ions. IV. Eu. *The Journal of Chemical Physics,* 1968, vol. 49 (10), 4450-4455
- **Mullin, J.W.** Particle size analysis with micro sieves using ultrasonic vibrations. *Chemistry and Industry,* 1971, 1435-1436
- **Rawle, A.** *Basic principles of particle size analysis,* 26 May 2003, http://www.malvern.co.uk/malvern/kbase.nsf/0/5E3F5A148D336B0480256 BF2006E2195/$file/Basic principles of particle size analysis MRK034-low res.pdf
- **Kumar, A ; Larsson, O ; Parodi, D ; Liang Z.** Silanised nucleic acids: a general platform for DNA immobilization. *Nucleic Acids Research Methods Online,* 2000, vol. 28 (14), 71